# EUROPEAN PATENT SPECIFICATION

(11) **EP 2 683 359 B1**
(45) Date of publication and mention of the grant of the patent: **22.04.2015**
(21) Application number: 12709232.8
(22) Date of filing: 06.03.2012
(51) Int. Cl.: A61K 9/00, A61M 37/00

(54) **MICRONEEDLE DEVICES AND METHODS**
MIKRONADELVORRICHTUNGEN UND VERFAHREN
DISPOSITIFS ET PROCÉDÉS DE MICRO-AIGUILLE

(30) Priority: 07.03.2011 US 201161449988 P
(43) Date of publication of application: 15.01.2014
(73) Proprietor: 3M Innovative Properties Company, Saint Paul, MN 55133-3427 (US)
(72) Inventor: ZHANG, Ying, Saint Paul, Minnesota 55133-3427 (US); HANSEN, Kristen J., Saint Paul, Minnesota 55133-3427 (US); DETERMAN, Amy S., Saint Paul, Minnesota 55133-3427 (US)
(74) Representative: Vossius & Partner Patentanwälte Rechtsanwälte mbB
(86) International application number: PCT/US2012/027858
(87) International publication number: WO 2012/122163

(56) References cited:
- US-A1- 2008 213 461
- US-A9- 2007 269 465
- US-B1- 6 562 363
- DATABASE WPI Week 200755 Thomson Scientific, London, GB; AN 2007-563309 XP002681423, -& JP 2007 089792 A (NANODEVICE SYSTEM KENKYUSHO KK) 12 April 2007 (2007-04-12)

## Description

### BACKGROUND

Transdermal delivery of a therapeutic agent such as a drug through the skin to the local tissue or systemic circulatory system without piercing the skin, such as with a transdermal patch, has been used successfully with certain agents. Passive delivery of this type involves the agent diffusing across at least the stratum corneum, where the rate of diffusion through the stratum corneum can be rate limiting.

In some instances, active delivery of a therapeutic agent has been conducted in order to increase agent flux through the stratum corneum. Here an external energy source, such as an electrical potential, ultrasound, or heat, is applied, thereby aiding the transport of the agent through the stratum corneum or through the skin.

Mechanically penetrating or disrupting the outermost skin layers in order to enhance the amount of agent being transdermally delivered has also been done. For example, during or after scratching the skin, such as with a scarifier, vaccines have been applied either topically of via the scarifier tines. In this case, the delivered amount is not critical, as only a very small amount of vaccine is needed to effectively immunize a patient.

Delivery of a desired amount of an agent by mechanically penetrating the stratum corneum can be compromised because of the mechanical and surface properties of skin. For example, skin can deflect and resist puncturing by very small piercing elements, causing non-uniform penetration of the skin. In addition, a coating on the piercing elements can be at least partially wiped from the element during penetration, and thereby fail to be deposited beneath the stratum corneum.

Use of an agent reservoir in communication with channels running through the piercing elements has been employed. The reservoir is pressurized in order to force the agent in fluid form through the small channels. Such systems are significantly more expensive to manufacture.

Microneedle devices having a dried coating on the surface of a microneedle array have desirable features compared to fluid reservoir devices. The devices are generally simpler and may directly introduce a therapeutic substance into the skin without the need for providing reliable control of fluid flow through very fine channels in the microneedle device.

Even with these developments, there continues to be an interest in and need for more predictable and controlled delivery of agents across the stratum corneum.

### SUMMARY

Microneedle devices, comprising solid microneedles coated with or containing certain local anesthetics in solid form, have now been made, which provide a controlled, immediate, and sustained dose of the local anesthetic to tissue underlying the stratum corneum, such as the epidermis.

Clinical procedures, including, for example, venepuncture, intravenous catheterization, and dermatological procedures, may cause pain or discomfort. In some instances, this has been addressed using topical anesthesia, such as EMLA™ cream, a eutectic mixture of 2.5% lidocaine and 2.5% prilocaine. However, minimum application time for these materials is on the order of 60 minutes.

Document JP 2007089792 discloses patches with coated microneedles. The coating comprises lidocaine and maltose. Document US 2008213461 discloses a device with dip-coated microneedles. The coating comprises lidocaine and PEG.

It has now been found that lidocaine and/or prilocaine tissue levels after only 1 minute exposure to the presently provided array of microneedles can be higher than the total level of lidocaine and prilocaine in tissue after a 60 minute application of EMLA™ cream. Moreover, unexpectedly, it has now been found that microneedles, coated with or containing lidocaine and/or prilocaine in combination with a local anesthetic dose-extending component selected from the group consisting of tetracaine, ropivacaine, bupivacaine, procaine and a combination thereof, can provide higher tissue levels of lidocaine and/or prilocaine for an extended period of time compared with lidocaine and/or prilocaine used without the dose-extending component. The local anesthetic dose is, therefore, found to be extended, or in other words, sustained at a higher level for a longer period of time.

In addition, the dose is limited to the amount of local anesthetic coated on or contained in the microneedles. After penetrating the stratum corneum, the local anesthetic in the coating on the microneedles dissolves in the tissue underlying the stratum corneum. In the case of dissolvable microneedles, the local anesthetic in the microneedles dissolves in the tissue. As such, the dose may be controlled without fear of delivering more than is needed or toxic levels.

Accordingly, in one embodiment there is provided a medical device, comprising:
an array of microneedles, and
a coating disposed on the microneedles,
wherein the coating comprises:
   a local anesthetic selected from the group consisting of lidocaine, prilocaine, and a combination thereof; and
   a local anesthetic dose-extending component selected from the group consisting of tetracaine, ropivacaine, bupivacaine, procaine and a combination thereof;
   wherein the local anesthetic is present in an amount of at least 1 wt-% based upon total weight of solids in the coating, and
   wherein the local anesthetic and dose-extending component are in a non-eutectic weight ratio.

In another embodiment, there is provided a method of extending a topically delivered local anesthetic dose in mammalian tissue, the method comprising:
contacting the tissue with a local anesthetic-coated microneedle device,
wherein the device comprises:
   an array of microneedles, and
   a coating disposed on the microneedles,
   wherein the coating comprises:
      a local anesthetic selected from the group consisting of lidocaine, prilocaine, and a combination thereof; and
      a local anesthetic dose-extending component selected from the group consisting of tetracaine, ropivacaine, bupivacaine, procaine and a combination thereof;
      wherein the local anesthetic is present in an amount of at least 1 wt-% based upon total weight of solids in the coating, and
      wherein the local anesthetic and dose-extending component are in a non-eutectic weight ratio.

In another embodiment, there is provided a method of making a local anesthetic-coated microneedle device comprising:
providing an array of microneedles,
providing a composition comprising:
   a local anesthetic selected from the group consisting of lidocaine, prilocaine, and a combination thereof;
   a local anesthetic dose-extending component selected from the group consisting of tetracaine, ropivacaine, bupivacaine, procaine and a combination thereof; and
   a volatilizable carrier;
contacting the microneedles with the composition, and
volatilizing at least a portion of the carrier to provide a coating disposed on the microneedles;
wherein the coating comprises the local anesthetic in an amount of at least 1 wt-% based upon total weight of solids in the coating, wherein the local anesthetic and dose-extending component are in a non-eutectic weight ratio; and
wherein the device comprises the array of microneedles with the coating disposed on the microneedles.

In another embodiment, there is provided a medical device, comprising an array of dissolvable microneedles, the microneedles comprising:
a dissolvable matrix material;
at least 1 wt-% of a local anesthetic selected from the group consisting of lidocaine, prilocaine, and a combination thereof; and
a local anesthetic dose-extending component selected from the group consisting of tetracaine, ropivacaine, bupivacaine, procaine and a combination thereof;
wherein the local anesthetic and dose-extending component are in a non-eutectic weight ratio, and
wherein wt-% is based upon total weight of solids in all portions of the dissolvable microneedles which contain the local anesthetic.

In another embodiment, there is provided a method of extending a topically delivered local anesthetic dose in mammalian tissue, the method comprising:
contacting the tissue with a local anesthetic-containing dissolvable microneedle device, wherein the device comprises an array of dissolvable microneedles comprising:
   a dissolvable matrix material;
   at least 1 wt-% of a local anesthetic selected from the group consisting of lidocaine, prilocaine, and a combination thereof; and
   a local anesthetic dose-extending component selected from the group consisting of tetracaine, ropivacaine, bupivacaine, procaine and a combination thereof;
   wherein the local anesthetic and the dose-extending component are in a non-eutectic weight ratio, and
   wherein wt-% is based upon total weight of solids in all portions of the dissolvable microneedles which contain the local anesthetic.

In another embodiment, there is provided a method of making a local anesthetic-containing dissolvable microneedle device, the method comprising:
providing a composition comprising a local anesthetic selected from the group consisting of lidocaine, prilocaine, and a combination thereof; a local anesthetic dose-extending component selected from the group consisting of tetracaine, ropivacaine, bupivacaine, procaine and a combination thereof; and a volatilizable carrier;
providing a mold having an array of microstructured cavities;
loading the composition into the mold;
volatilizing at least a portion of the volatilizable carrier;
providing a composition comprising a dissolvable matrix material and a volitilizable carrier;
loading the composition comprising the dissolvable matrix material into the mold;
volatilizing at least a portion of the volatilizable carrier; and
removing a solid dissolvable microneedle array comprising dissolvable microneedles from the mold;
wherein the dissolvable microneedles comprise at least 10 wt-% dissolvable matrix material, at least 1 wt-% local anesthetic, and the dose-extending component; wherein the local anesthetic and dose-extending component are in a non-eutectic weight ratio; and
   wherein wt-% is based upon total weight of solids in all portions of the dissolvable microneedles which contain the local anesthetic; and
wherein the device comprises the solid dissolvable microneedle array.

In another embodiment, there is provided a method of making a local anesthetic-containing dissolvable microneedle device, the method comprising:
providing a composition comprising a dissolvable matrix material; a local anesthetic selected from the group consisting of lidocaine, prilocaine, and a combination thereof; a local anesthetic dose-extending component selected from the group consisting of tetracaine, ropivacaine, bupivacaine, procaine and a combination thereof; and a volatilizable carrier;
providing a mold having an array of microstructured cavities;
loading the composition into the mold;
volatilizing at least a portion of the volatilizable carrier; and
removing a solid dissolvable microneedle array comprising dissolvable microneedles from the mold;
wherein the dissolvable microneedles comprise at least 10 wt-% dissolvable matrix material, at least 1 wt-% local anesthetic, and the dose-extending component; wherein the local anesthetic and dose-extending component are in a non-eutectic weight ratio; and wherein wt-% is based upon total weight of solids in all portions of the dissolvable microneedles which contain the local anesthetic; and
wherein the device comprises the solid dissolvable microneedle array.

### DEFINITIONS

The following terms are used herein according to the following definitions.

The term "wt-%" means weight percent. In embodiments where wt-% is based upon total weight of solids, solids are those ingredients which are not volatile. For example, the total weight of solids does not include the volatilizable carrier.

The term "volatilizable carrier" refers to materials which can be volatilized and in which the local anesthetic and dose-extending component may be dissolved or dispersed. Such materials include, for example, water and/or volatile organic solvents, such as, for example, short chain alcohols, short chain ethers, short chain ketones, and short chain esters (e.g., C₁₋₄ monohydroxy alcohols, C₁₋₄ ethers, C₁₋₄ ketones, C₁₋₄ esters, and the like).

Material which can be volatilized are those wherein at least 50 percent of the material volatilizes from a coating on the microneedles at an ambient temperature and duration at which less than 1 percent of the local anesthetic and dose-extending component degrade. For certain embodiments, the volatilizable carrier has a boiling point of at most 120 °C, preferably at most 100 °C.

"Subject" and "patient" include humans, sheep, horses, cattle, pigs, dogs, cats, rats, mice, or other mammals.

The terms "comprises" and variations thereof do not have a limiting meaning where these terms appear in the description and claims.

The above summary of the present invention is not intended to describe each disclosed embodiment or every implementation of the present invention. The description that follows more particularly exemplifies illustrative embodiments. In the application, guidance is provided through lists of examples, which examples can be used in various combinations. In each instance, the recited list serves only as a representative group and should not be interpreted as an exclusive list.

### BRIEF DESCRIPTION OF THE DRAWINGS

The disclosure may be more completely understood in consideration of the following detailed description of various embodiments of the disclosure in connection with the accompanying drawings, in which:
**FIG. 1** is a schematic cross-sectional view of an uncoated microneedle array.
**FIG. 2** is a schematic perspective view of a microneedle device in the form of a patch.
**FIG. 3** is a schematic cross-sectional view of a coated microneedle array.
**FIG. 4** is a schematic cross-sectional view of a dissolvable microneedle array.
**FIG. 5** is an optical micrograph of uncoated microneedles in a microneedle array.
**FIG. 6** is an optical micrograph of coated microneedles in a microneedle array.
**FIG. 7** is an optical micrograph of coated microneedles in a microneedle array after 1 minute in tissue.

The figures are not necessarily to scale. Like numbers used in the figures refer to like components. However, it will be understood that the use of a number to refer to a component in a given figure is not intended to limit the component in another figure labeled with the same number.

### DETAILED DESCRIPTION OF ILLUSTRATIVE EMBODIMENTS

In the following description, reference is made to the accompanying drawings that form a part hereof, and in which are shown by way of illustration several specific embodiments. It is to be understood that other embodiments are contemplated and may be made without departing from the scope or spirit of the present disclosure. The following detailed description, therefore, is not to be taken in a limiting sense.

All scientific and technical terms used herein have meanings commonly used in the art unless otherwise specified. The definitions provided herein are to facilitate understanding of certain terms used frequently herein and are not meant to limit the scope of the present disclosure.

Unless otherwise indicated, all numbers expressing feature sizes, amounts, and physical properties used in the specification and claims are to be understood as being modified in all instances by the term "about." Accordingly, unless indicated to the contrary, the numerical parameters set forth in the foregoing specification and attached claims are approximations that can vary depending upon the desired properties sought to be obtained by those skilled in the art utilizing the teachings disclosed herein.

The recitation of numerical ranges by endpoints includes all numbers subsumed within that range (e.g., 1 to 5 includes 1, 1.5, 2, 2.75, 3, 3.80, 4, and 5) and any range within that range.

As used in this specification and the appended claims, the singular forms "a", "an", and "the" encompass embodiments having plural referents, unless the content clearly dictates otherwise. As used in this specification and the appended claims, the term "or" is generally employed in its sense including "and/or" unless the content clearly dictates otherwise.

As indicated above, provided herein are medical devices which can deliver a local anesthetic to tissue of a subject, methods of using the devices, and methods of making the devices. The devices include an array of microneedles which are either coated with the local anesthetic or which are dissolvable and contain the local anesthetic.

The local anesthetic, which can be lidocaine, prilocaine, or a combination thereof, is combined with the dose-extending component. The dose-extending component/local anesthetic weight ratio is non-eutectic. In a non-eutectic weight ratio the dose-extending component/local anesthetic combination is in solid form. In the present devices and methods, the solid form is found to be more stable and provides a more reproducible delivery than a liquid form. To achieve this in instances where a eutectic mixture is possible, the weight ratio is preferably other than 1:1. For example, the free base forms of tetracaine and lidocaine in a 1:1 ratio form a liquid (eutectic mixture), which is avoided by using a non-eutectic weight ratio.

As used herein, the local anesthetic and dose-extending component include the free base thereof, a pharmaceutically acceptable salt thereof, and/or a combination of free base and pharmaceutically acceptable salt. The local anesthetic weight and dose-extending component weight as well as the dose-extending component/local anesthetic weight ratio may be calculated based upon the weight of local anesthetic and the weight of dose-extending component used or, alternatively, upon the weights of the free base forms of the local anesthetic and dose-extending component used. In this alternative case, for example, if a salt is used, the weight of the anion portion is subtracted out to give the weight of the free base form.

For certain embodiments, including any one of the above embodiments, the dose-extending component/local anesthetic weight ratio is preferably at least 0.1, more preferably at least 0.3, even more preferably at least 0.5. For certain of these embodiments, the weight ratio is preferably at most 0.9, more preferably at most 0.8. Alternatively, the weight ratio is at most 3, preferably at most 2.5, 2.0 or 1.5. For certain embodiments, including any one of the above embodiments, the dose-extending component/local anesthetic weight ratio is 0.1 to 0.9.

For certain embodiments, including any one of the above embodiments, preferably the local anesthetic is present in an amount of at least 1 wt-% based upon total weight of solids in the coating, more preferably at least 3 wt-%, more preferably at least 5 wt-%, more preferably at least 10 wt-%, most preferably at least 20 wt-%. For certain of these embodiments, the local anesthetic is present in an amount of at most 90 wt-% based upon total weight of solids in the coating, preferably at most 80 wt-%, more preferably at most 70 wt-%. For certain embodiments, including any one of the above embodiments, the local anesthetic is present in an amount of 20 wt-% to 90 wt-%, based upon total weight of solids in the coating.

For certain embodiments, including any one of the above embodiments, the dose-extending component is present in an amount of at least 0.1 wt-% based upon the total weight of solids in the coating. For certain of these embodiments, preferably the dose-extending component is present in an amount of at least 1 wt-%, more preferably at least 2 wt-%, most preferably at least 5 wt-% or 10 wt-%. For certain of these embodiments, the dose-extending component is present in an amount of at most 75 wt-%, preferably at most 60 wt-%, more preferably at most 50 wt-% or 40 wt-%. For certain embodiments, including any one of the above embodiments, the dose-extending component is present in an amount of 2 wt-% to 48 wt-%, based upon total weight of solids in the coating.

As indicated above, the local anesthetic dose-extending component is selected from the group consisting of tetracaine, ropivacaine, bupivacaine, procaine and a combination thereof. For certain embodiments, including any one of the above embodiments, the dose-extending component is selected from the group consisting of tetracaine, procaine, bupivacaine, and a combination thereof. For certain of these embodiments, the dose-extending component is tetracaine.

The present coatings and dissolvable microneedles may also include at least one excipient. An excipient can function to maintain the active nature of the local anesthetic and the dose-extending component, to facilitate the performance of a coating formulation when depositing a coating on the microneedles, to resist disruption of the coating when penetrating the stratum corneum or other tissue, or a combination thereof. Accordingly, for certain embodiments, including any one of the above embodiments which includes a coating deposited on microneedles or the microneedle itself comprising the local anesthetic, the coating or microneedle itself further comprises at least one excipient.

The amount of the at least one excipient in the coating, and therefore in the coating formulation used for depositing the coating can vary depending on the identity of the components in the coating formulation, the amount of local anesthetic and dose-extending component desired on the microneedle array, the type of microneedle array being coated, the shape and location of the coating on the microneedle, other considerations not discussed herein, or some combination thereof.

For certain embodiments, including any one of the above embodiments which includes an excipient, preferably the excipient is present in the coating in an amount of at least 2 wt-% based upon the total weight of solids in the coating, more preferably at least 5 wt-%, most preferably at least 10 wt-%. For certain of these embodiments, preferably the exipient is present in the coating in an amount of at most 98 wt-%, more preferably at most 90 wt-%, most preferably at most 75 wt-% or 50 wt-%. For certain of these embodiments, preferably the coating comprises 10 to 75 wt-% or 10 to 50 wt-% of the at least one excipient, wherein wt-% is based upon total solids content of the coating.

Exemplary excipients can include, for example, buffers, carbohydrates, polymers, amino acids, peptides, surfactants, proteins, non-volatile non-aqueous solvents, acids, bases, antioxidants and saccharin.

At least one buffer may be used for at least a portion of the at least one excipient. The buffer can generally function to stabilize the pH of a coating formulation used for depositing the coating on the microneedles. The particular buffer to be utilized can depend at least in part on the particular local anesthetic and the dose-extending component that are included in the coating. The pH of the formulation can, for example, help to maintain the solubility of the local anesthetic and dose-extending component at a desired level. Generally, commonly utilized buffers can be used in the coating formulations.

Exemplary buffers can include for example, histidine, phosphate buffers, acetate buffers, citrate buffers, glycine buffers, ammonium acetate buffers, succinate buffers, pyrophosphate buffers, Tris acetate (TA) buffers, and Tris buffers. Buffered saline solutions can also be utilized as buffers. Exemplary buffered saline solutions include, for example, phosphate buffered saline (PBS), Tris buffered saline (TBS), saline-sodium acetate buffer (SSA), saline-sodium citrate buffer (SSC).

At least one carbohydrate, including mixtures of carbohydrates, may be used for at least a portion of the at least one excipient. The carbohydrate can be a saccharide, including mono-, di-, and polysaccharides, and may include, for example, non-reducing sugars such as raffinose, stachyose, sucrose, and trehalose; and reducing sugars such as monosaccharides and disaccharides. Exemplary monosacharides can include apiose, arabinose, digitoxose, fucose, fructose, galactose, glucose, gulose, hamamelose, idose, lyxose, mannose, ribose, tagatose, sorbitol, xylitol, and xylose. Exemplary disaccharides can include for example sucrose, trehalose, cellobiose, gentiobiose, lactose, lactulose, maltose, melibiose, primeverose, rutinose, scillabiose, sophorose, turanose, and vicianose. In embodiments, sucrose, trehalose, fructose, maltose, or combinations thereof can be utilized. All optical isomers of exemplified sugars (D, L, and racemic mixtures) are also included herein.

Polysaccharides can include for example starches such as hydroxyethyl starch, pregelatinized corn starch, pentastarch, dextrin, dextran or dextran sulfate, gamma-cyclodextrin, alpha-cyclodextrin, beta-cyclodextrin, glucosyl-alpha-cyclodextrin, maltosyl-alpha-cyclodextrin, glucosyl-beta-cyclodextrin, maltosyl-beta-cyclodextrin, 2-hydroxy-beta-cyclodextrin, 2-hydroxypropyl-beta-cyclodextrin, 2-hydroxypropyl-gamma-cyclodextrin, hydroxyethyl-beta-cyclodextrin, methyl-beta-cyclodextrin, sulfobutylether-alpha-cyclodextrin, sulfobutylether-beta-cyclodextrin, and sulfobutylether-gamma-cyclodextrin. In embodiments, hydroxyethyl starch, dextrin, dextran, gamma-cyclodextrin, beta-cyclodextrin, or combinations thereof can be utilized. In embodiments, dextrans having an average molecular mass of 35,000 to 76,000 can be utilized.

The at least one carbohydrate can be a cellulose. Suitable celluloses can include for example hydroxyethyl cellulose (HEC), methyl cellulose (MC), microcrystalline cellulose, hydroxypropyl methyl cellulose (HPMC), hydroxyethylmethyl cellulose (HEMC), hydroxypropyl cellulose (HPC), and mixtures thereof.

At least one polymer may be used for at least a portion of the at least one excipient. Suitable polymers include, for example, polyvinyl pyrrolidone (PVP), polyethylene glycol (PEG), polyvinyl alcohol (PVA), and polyethylene glycol sorbitan isostearate. In embodiments, polyvinyl pyrrolidones (PVP) having an average molecular weight of 10,000 can be utilized. In embodiments, polyvinyl pyrrolidones (PVP) having an average molecular weight of 5,000 to 1.5 million can be utilized. In embodiments, polyethylene glycols having an average molecular weight of 300 to 8,000 can be utilized.

At least one amino acid may be used for at least a portion of the at least one excipient. Suitable amino acids can include for example lysine, histidine, cysteine, glutamate, lysine acetate, sarcosine, proline, threonine, asparagine, aspartic acid, glutamic acid, glutamine, isoleucine, leucine, methionine, phenylalanine, serum tryptophan, tyrosine, valine, alanine, arginine, and glycine. In many cases the salt form of the amino acids can be used to increase the aqueous solubility of the amino acid in an aqueous media or formulation.

At least one peptide may be used for at least a portion of the at least one excipient. The amino acids making up the peptide may be the same or at least some may be different from each other. Suitable polyamino acids (the same amino acids) can include for example polyhistidine, polyaspartic acid, and polylysine.

At least one protein may be used for at least a portion of the at least one excipient. Suitable proteins can include for example human serum albumin and bioengineered human albumin.

At least one saccharin may be used for at least a portion of the at least one excipient. In one example, the saccharin is saccharin sodium dihydrate.

At least one lipid may be used for at least a portion of the at least one excipient. In one example, the lipid may be dipalmitoylphosphatidylcholine (DPPC).

At least one acid and/or base may be used for at least a portion of the at least one excipient. For example, at least one weak acid, weak base, strong acid, strong base, or some combination thereof may be used. Acids and bases can serve the purpose of solubilizing or stabilizing the local anesthetic and/or the dose-extending compnent. These acids and bases can be referred to as counterions. These acids and bases can be organic or inorganic. Exemplary weak acids include for example acetic acid, propionic acid, pentanoic acid, citric acid, succinic acid, glycolic acid, gluconic acid, glucuronic acid, lactic acid, malic acid, pyruvic acid, tartaric acid, tartronic acid, fumaric acid, glutamic acid, aspartic acid, malonic acid, butyric acid, crotonic acid, digylcolic acid, and glutaric acid. Exemplary strong acids include for example hydrochloric acid, hydrobromic acid, nitric acid, sulfonic acid, sulfuric acid, maleic acid, phosphoric acid, benzene sulfonic acid, and methane sulfonic acid. Exemplary weak bases include for example ammonia, morpholine, histidine, lysine, arginine, monoethanolamine, diethanolamine, triethanolamine, tromethamine, methylglucamine, and glucosamine. Exemplary strong bases include for example sodium hydroxide, potassium hydroxide, calcium hydroxide, and magnesium hydroxide.

At least one surfactant may be used for at least a portion of the at least one excipient. The at least one surfactant can be amphoteric, cationic, anionic, or nonionic. Suitable surfactants can include for example lecithin, polysorbates (such as polysorbate 20, polysorbate 40, and polysorbate 80 for example), glycerol, sodium lauroamphoacetate, sodium dodecyl sulfate, cetylpyridinium chloride (CPC), dodecyltrimethyl ammonium chloride (DoTAC), sodium desoxycholate, benzalkonium chloride, sorbitan laurate, and alkoxylated alcohols (such as laureth-4).

At least one inorganic salt may be used for at least a portion of the at least one excipient. Suitable inorganic salts can include for example sodium chloride, and potassium chloride.

A non-volatile, non-aqueous solvent may also be used for at least a portion of the at least one excipient. Examples may include propylene glycol, dimethylsulfoxide, glycerin, 1-methyl-2-pyrrolidinone, *N*,*N*-dimethylformamide, and the like.

At least one antioxidant may be used for at least a portion of the at least one excipient. Suitable antioxidants can include for example sodium citrate, citric acid, ascorbic acid, methionine, sodium ascorbate, and combinations thereof.

For certain embodiments, including any one of the above embodiments which includes an excipient, preferably the at least one excipient is selected from the group consisting of sucrose, dextrins, dextrans, hyroxyethyl cellulose (HEC), polyvinyl pyrrolidone (PVP), polyethylene glycols, amino acids, peptides, polysorbates, human serum albumin, saccharin sodium dihydrate, and a combination thereof.

For certain embodiments, including any one of the above embodiments which includes an excipient, the at least one excipient is a saccharide. For certain of these embodiments, the saccharide is selected from the group consisting of dextran, sucrose, trehalose, and a combination thereof.

As indicated above, in the method of making a local anesthetic-coated microneedle device provided herein, a composition is provided which includes a local anesthetic selected from the group consisting of lidocaine, prilocaine, and a combination thereof; a local anesthetic dose-extending component selected from the group consisting of tetracaine, ropivacaine, bupivacaine, procaine and a combination thereof; and a volatilizable carrier; wherein the dose-extending component/local anesthetic weight ratio is non-eutectic. The amounts of these ingredients in the composition are chosen in order to achieve the above described amounts of the solid, non-volatile ingredients in the resulting coating deposited on the microneedles. This composition is also referred to herein as a coating formulation and may further include any of the excipients as described above and amounts thereof in order to achieve the amounts in the deposited coating as described above. The coating is deposited on the microneedles by contacting the microneedles with the composition.

Coating formulations used for depositing the coating on the microneedles generally include water as a solvent, which is a volatilizable carrier. Generally, the solvent in the coating formulation is selected such that it may dissolve or disperse the local anesthetic, the dose-extending component, and any excipients, if present. The coating formulation can also include at least one co-solvent (which may also be a volatilizable carrier) in addition to water. Examples of volatile co-solvents (also volatilizable carriers) which may be used include ethanol, isopropanol, methanol, propanol, and butanol. Examples of non-volatile co-solvents, also referred to above as excipients, include propylene glycol, dimethysulfoxide, glycerin, 1-methyl-2-pryrrolidinone, and *N,N-*dimethylformamide.

For certain embodiments, preferably the coating formulations can have an overall solids content from 5% to 80% by weight; from 10% to 70% by weight; or from 50% to 70% by weight.

Coating formulations used for depositing the coating on the microneedles can be further described by various properties of the formulations. Exemplary properties that can be utilized to further describe the coating formulations include for example, the viscosity of the coating formulation, the surface tension of the coating formulation, the contact angle of the coating formulation on the material comprising the microneedles, or some combination thereof.

Generally, viscosity is a measurement of the resistance of a fluid which is being deformed by either shear stress or tensile stress. Coating formulations can be characterized by their resistance to being deformed by a shear stress, which can also be referred to as the shear viscosity of the aqueous formulation. Various instruments can be used for viscosity testing, including rheometers, for example rheometers from TA Instruments (New Castle, DE).

When a coating formulation is too viscous, the coating formulation will be difficult to utilize in manufacturing methods, can produce non-reproducible coatings (and therefore non-reproducible amounts of local anesthetic and dose-extending component that will be administered by the microneedle array upon use), and can result in an overall reduction in the coating weight. If a coating formulation is not viscous enough, the coating formulation will not be able to effectively coat the microneedle surfaces (which could therefore require more dips of the microneedle in the coating formulation, thereby increasing the manufacturing costs), and in some cases capillary forces can cause the formulation to coat the microneedle and the microneedle substrate, which is sometimes referred to as "capillary jump". The desired viscosity of a coating formulation can depend at least in part on the geometry of the microneedles, the particular coating method being utilized, the desired number of coating steps, other considerations not discussed herein, or some combination thereof.

For certain embodiments, including any one of the above method embodiments which includes contacting the microneedles with the composition, preferably the coating formulation can have a viscosity (or shear viscosity) of from 500 to 30,000 centipoise (cps) when measured at a shear rate of 100s⁻¹ at a temperature of 25° C, more preferably 500 to 10,000 cps, even more preferably from 500 to 8,000 cps.

Various methods can be utilized to measure surface tension of the coating formulation. An exemplary type of surface tension measurement is based on the pendant drop method. In a pendant drop method of measuring surface tension, a drop of liquid is suspended from the end of a tube by surface tension. The force due to surface tension is proportional to the length of the boundary between the liquid and the tube. Various instruments that encompass optical systems for measuring the relevant parameters of the drop and software packages for calculating the surface tension based on the measured parameters can be utilized herein. An exemplary instrument includes the Drop Shape Analysis System (Model DSA 100S) available from Krüss (Hamburg, Germany).

Generally, if a coating formulation has too high a surface tension, the coating formulation may not be able to effectively coat the microneedle surfaces (which could therefore require more dips of the microneedle in the coating formulation thereby increasing the manufacturing costs), it may be difficult to get the coating formulation to effectively coat the microneedle, or a combination thereof. If a coating formulation has too low a surface tension, the coating formulation may spread excessively along the needle due to "favorable wetting of the surface", in which case it not only coats the tip of the microneedle but it extends further down the microneedle toward the microneedle substrate and may in some cases actually coat the microneedle substrate. The desired surface tension of a coating formulation can depend at least in part on the geometry of the microneedles, the particular coating method being utilized, the desired number of coating steps, other considerations not discussed herein, or some combination thereof.

For certain embodiments, including any one of the above method embodiments which includes contacting the microneedles with the composition (coating formulation), preferably the composition can have a surface tension (measured at ambient, or room temperature conditions) that is not greater than 60 dynes/cm, more preferably not greater than 55 dynes/cm. For certain of these embodiments, preferably the coating formulation has a surface tension from 40 dynes/cm to 55 dynes/cm.

A coating formulation can be further characterized by its contact angle with the material comprising the microneedles (also referred to as the "microneedle material"). It should be noted that the contact angle of the coating formulation with respect to the microneedle material is measured on a horizontal substrate made of the microneedle material.

The microneedle material can be (or include) silicon or a metal such as stainless steel, titanium, or nickel titanium alloy. The microneedle material can also be (or include) a medical grade polymeric material. Generally, the contact angle of a coating formulation with the microneedle material is an indication of the affinity of the coating formulation for the microneedle material. The lower the contact angle is, the stronger the attraction of the coating formulation for the microneedle material, resulting in increased wetting of the microneedle surface. The contact angle of the coating formulation on the microneedle material can be measured using various methods, for example, using the sessile drop method. Generally, a goniometer (or an instrument that employs a goniometer) can be utilized to measure contact angles, an example of such an instrument is the Drop Shape Analysis System (Model DSA 100S) available from Krüss (Hamburg, Germany). In embodiments, the contact angle can be measured within 5 seconds of the transfer of the coating formulation onto the substrate (microneedle material).

Generally, if a coating formulation has a contact angle that is too low (the coating formulation is strongly attracted to the microneedle material), the coating formulation can produce inconsistent coatings (and therefore inconsistent amounts of local anesthetic and dose-extending component on the microneedle array), or the coating formulation may spread excessively along the needle due to "favorable wetting of the surface", in which case it not only coats the tip of the microneedle but it extends further down the microneedle toward the microneedle substrate and may in some cases actually coat the microneedle substrate. A contact angle that is too low can also increase the chances of capillary jump, particularly in a coating formulation having a low viscosity. If a coating formulation has a contact angle that is too high (the coating formulation is not strongly attracted or even repelled from the microneedle material), it may be difficult to get the coating formulation to effectively coat the microneedle. The desired contact angle of a coating formulation on the microneedle material can depend at least in part on the composition of the microneedles, geometry of the microneedles, the particular coating method being utilized, the desired number of coating steps, other considerations not discussed herein, or some combination thereof.

For certain embodiments, including any one of the above method embodiments which includes contacting the microneedles with the composition, preferably the composition (coating formulation) can have a contact angle (measured at ambient, or room temperature conditions) with the microneedle material of 50 degrees or greater, 55 degrees or greater, or 65 degrees or greater.

For certain embodiments, including any one of the above embodiments, microneedle material can be a medical grade polymeric material. Exemplary types of medical grade polymeric materials include for example, polycarbonate, and liquid crystalline polymer (referred to herein as "LCP").

As indicated above, the method of making a local anesthetic-coated microneedle device provided herein includes a step of providing an array of microneedles. The step of providing the microneedle array can be accomplished by manufacturing the microneedle array, obtaining a microneedle array (for example by purchasing the microneedle array), or by some combination thereof.

Generally, an "array" refers to medical devices described herein that include more than one (in embodiments, a plurality) structure capable of piercing the stratum corneum to facilitate the transdermal delivery of the local anesthetic and dose-extending component to the skin. The terms "microstructure", or "microneedle" refer to the structures associated with an array that are capable of piercing the stratum corneum to facilitate the transdermal delivery of the local anesthetic and dose-extending component to the skin. By way of example, microstructures can include needle or needle-like structures as well as other structures capable of piercing the stratum corneum. The term "microneedle array" or "array of microneedles" therefore can refer to a plurality of structures that are capable of piercing the stratum corneum to facilitate the transdermal delivery of the local anesthetic and dose-extending component to the skin.

Microneedle arrays useful in disclosed embodiments may include any of a variety of configurations, such as those described in the following patents and patent applications, the disclosures of which are incorporated herein by reference thereto. One embodiment for the microneedle arrays includes the structures disclosed in U. S. Patent Application Publication No. 2005/0261631 which describes microneedles having a truncated tapered shape and a controlled aspect ratio. A further embodiment for the microneedle arrays includes the structures disclosed in U.S. Patent No. 6,881,203 , which describes tapered microneedles with at least one channel formed on the outside surface. Another embodiment for the microneedle arrays includes the structures disclosed in U.S. Provisional Patent Application 61/168,268 and U.S. Provisional Patent Application 61/115,840, which both describe hollow microneedles. For certain embodiments, including any one of the embodiments described herein, preferably the microneedles are solid microneedles. Solid microneedles are solid throughout.

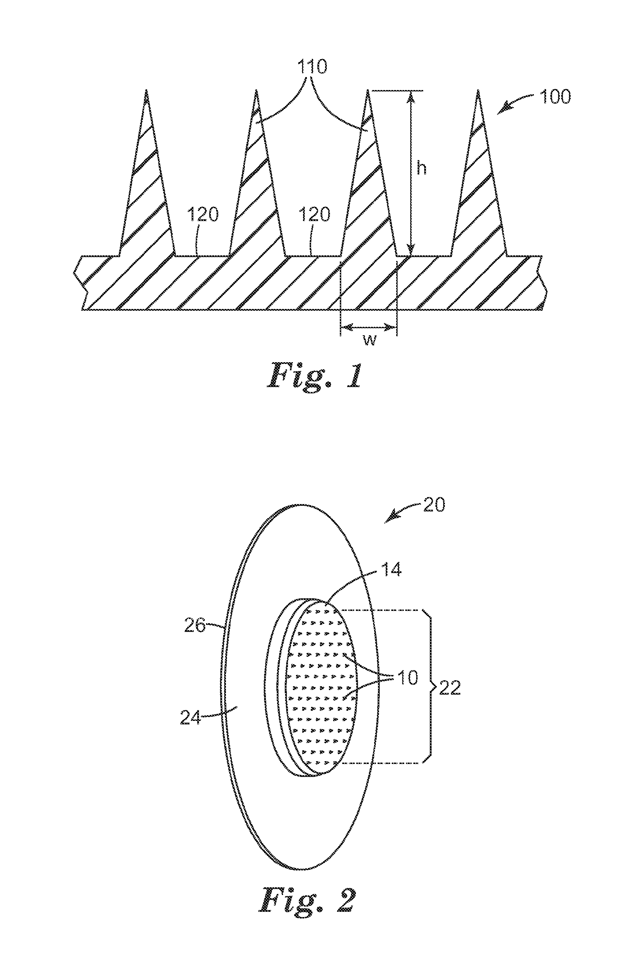

Generally, a microneedle array includes a plurality of microneedles. **FIG. 1** shows a portion of a microneedle array **100** that includes four microneedles **110** (of which two are referenced in **FIG. 1****)** positioned on a microneedle substrate **120.** Each microneedle **110** has a height **h,** which is the length from the tip of the microneedle **110** to the microneedle base at substrate **120.** Either the height of a single microneedle or the average height of all microneedles on the microneedle array can be referred to as the height of the microneedle, **h.** For certain embodiments, including any one of the embodiments described herein, each of the plurality of microneedles (or the average of all of the plurality of microneedles) have a height of about 100 to 1200 micrometers (µm), preferably about 200 to 1000 µm, more preferably about 200 to 750 µm. For certain embodiments, including any one of the embodiments described herein, the array of microneedles contains 200 to 1500 microneedles per cm² of the array of microneedles.

A single microneedle or the plurality of microneedles in a microneedle array can also be characterized by their aspect ratio. The aspect ratio of a microneedle is the ratio of the height of the microneedle, **h,** to the width (at the base of the microneedle), **w** (as seen in **FIG. 1****).** The aspect ratio can be presented as **h:w.** For certain embodiments, including any one of the embodiments described herein, each of the plurality of microneedles (or the average of all of the plurality of microneedles) has (have) an aspect ratio in the range of 2:1 to 5:1. For certain of these embodiments, each of the plurality of microneedles (or the average of all of the plurality of microneedles) has (have) an aspect ratio of at least 3:1.

A microneedle or the plurality of microneedles in a microneedle array can also be characterized by shape. For certain embodiments, including any one of the embodiments described herein, each of the plurality of microneedles can have a square pyramidal shape or the shape of a hypodermic needle. For certain of these embodiments, preferably the shape is square pyramidal.

For certain embodiments, including any one of the embodiments described herein, the device may be in the form of a patch. One example of such an embodiment is shown in more detail in **FIG. 2. FIG. 2** illustrates a device comprising a patch **20** in the form of a combination of a microneedle array **22,** pressure sensitive adhesive **24** and backing **26.** Such a patch **20,** or a device including multiple microneedle arrays or multiple patches **20** can be referred to as a delivery device. The microneedle array **22** is illustrated with microneedles **10** protruding from a microneedle substrate **14.** The microneedles **10** may be arranged in any desired pattern or distributed over the microneedle substrate **14** randomly. As shown, the microneedles **10** are arranged in uniformly spaced rows. For certain embodiments, including any one of the embodiments described herein, microneedle arrays can have a distal-facing surface area of more than about 0.1 cm² and less than about 20 cm². For certain of these embodiments, preferably the microneedle array area is more than about 0.5 cm² and less than about 5 cm². In one embodiment (not shown), a portion of the substrate **14** of the patch **20** is non-patterned. In one embodiment the non-patterned surface has an area of more than about 1 percent and less than about 75 percent of the total area of the device surface that faces a skin surface of a patient. In one embodiment the non-patterned surface has an area of more than about 0.10 square inch (0.65 cm²) to less than about 1 square inch (6.5 cm²). In another embodiment (shown in **FIG. 2****),** the microneedles are disposed over substantially the entire surface area of the array **22,** such that there is essentially no non-patterned area.

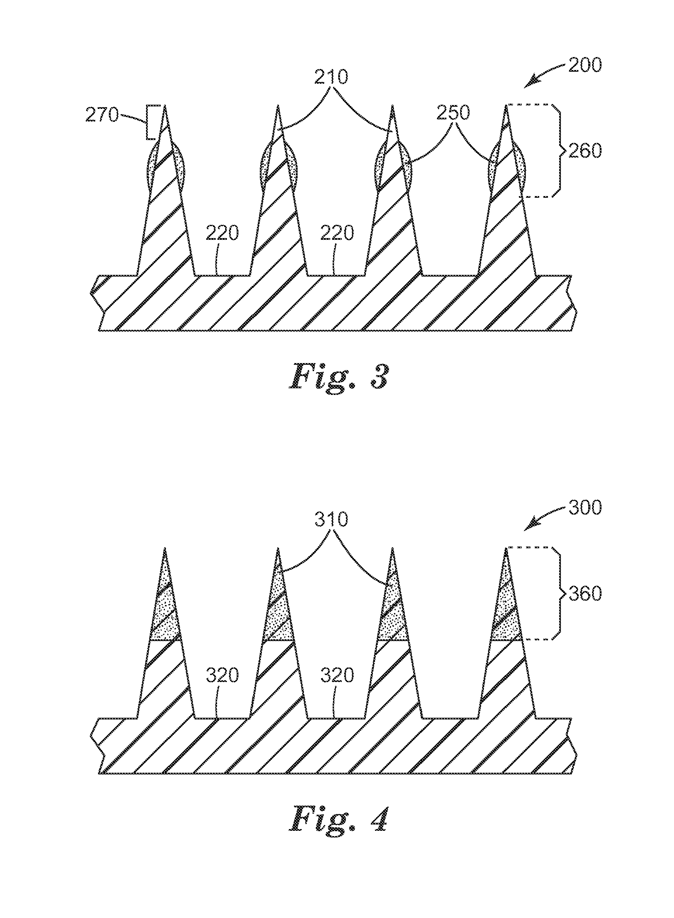

In the method of making a local anesthetic-coated microneedle device described herein, the step of contacting the microneedles with the composition (also referred to herein as the coating formulation) can be carried out by dip coating the microneedles. Such methods are described, for example, in copending U.S. Provisional Patent Application 61/349,317 filed May 28, 2010 , particularly with reference to FIGS. 3A, 3B, and 3C therein.

When dip coating, wasting local anesthetic and dose-extending component is avoided by contacting only a portion of the microneedle height with the coating formulation and avoiding contact with the microneedle substrate. **FIG. 3** illustrates, in cross-section, a portion of a microneedle array **200** that includes four microneedles **210** (of which two are referenced in **FIG. 3****)** positioned on a microneedle substrate **220.** Coating **250** is disposed on microneedles **210** no more than distance **260** from the tip of the microneedles. This is accomplished by contacting not more than a portion of the microneedle height with the coating formulation. Accordingly, for certain embodiments, including any one of the method embodiments described herein that includes the step of contacting the microneedles with the composition (also referred to herein as the coating formulation) the microneedles each have a tip and a base, the tip extending a distance **(h)** from the base, and contacting is carried out by contacting the tips of the microneedles and a portion of the microneedles extending not more than 90 percent of the distance (0.9**h**) from the tips to the bases with the composition, preferably not more than 70 percent of the distance (0.7**h**), more preferably not more than 50 percent of the distance (0.5**h**). It is to be understood that the distance can apply to a single microneedle or to an average of the microneedles in an array. For certain embodiments, including any one of the embodiments described herein which includes a coating disposed on the microneedles, at least 50 % of the microneedles have the coating present on the microneedles near the tip and extending not more than 90 percent of the distance toward the base, preferably not more than 70 percent of the distance, more preferably not more than 50 percent of the distance.

When the microneedles are contacted with the coating formulation, the microneedles are facing downward into the coating formulation. For certain embodiments, preferably after the microneedles are contacted with the coating formulation, contacting is terminated and the microneedles are positioned facing upward prior to and/or during volatilizing at least a portion of the volatilizable carrier. In this position, a portion of the coating formulation remaining on the microneedles may flow toward the base, leaving the tips of the microneedles exposed or with only a small amount of coating formulation on the tips. The degree to which flow occurs can depend upon factors such as the viscosity, contact angle, and surface tension as described above.

After removing the microneedles from the coating formulation, some of the coating formulation remains on the microneedles, the amount depending upon the coating formulation properties and surface properties of the microneedle material as described above. At least a portion of the volatilizable carrier is removed from the coating formulation adhering to the microneedles, leaving the coating disposed on the microneedles. One or more additional contacting steps may be used. The shape of the coating, average coating thickness, and amount of the surface of the microneedle covered by the coating depends upon the factors discussed above as well as the number of times the contacting step is repeated.

**FIG. 3** illustrates one embodiment with the coating disposed on the microneedles, wherein the tips of the microneedles are essentially exposed (no coating or a relatively small amount of coating) a distance **270** from the tip. For certain embodiments, including any one of the embodiments described herein which includes a coating disposed on the microneedles, the tips of the microneedles are exposed or only as small amount of coating is on the tips. For certain of these embodiments distance **270** is at least 1 percent (0.1**h**), 3 percent (0.03**h**) or 6 percent (0.06**h**) of the distance from the tip to the base. For certain of these embodiments, distance **270** is at most 10 percent (0.1**h**) of the distance from the tip to the base.

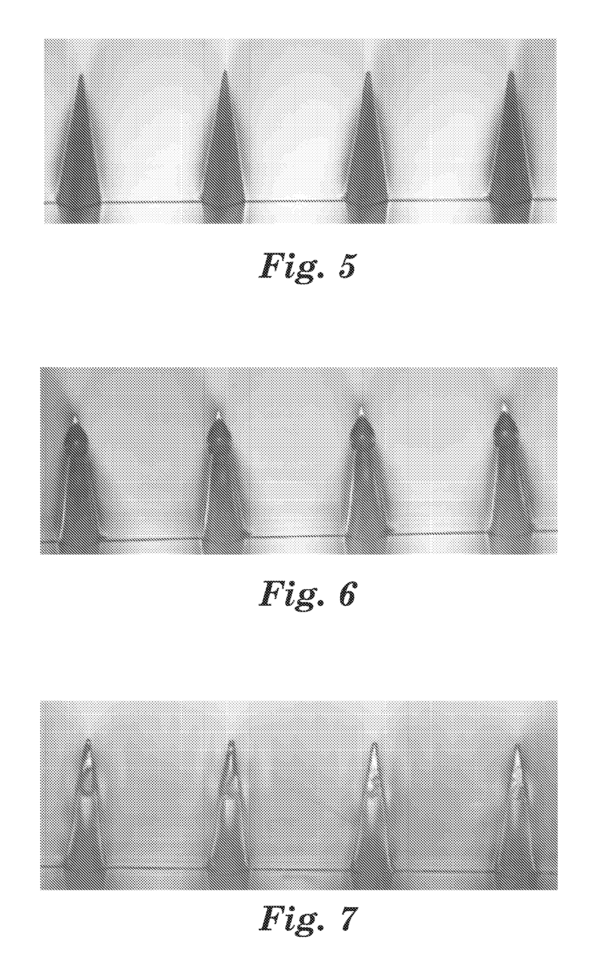

**FIG. 5** is an optical micrograph illustrating four microneedles of a microneedle array prior to contacting the microneedles with the composition (coating formulation).

For certain embodiments, including any one of the embodiments described herein which includes a coating disposed on the microneedles, the coating is present on the microneedles in an average amount of 0.01 to 2 micrograms per microneedle. Coating weight can be determined by weighing the microneedle array before and after the coating is disposed on the microneedles and dividing the difference by the number of microneedles in the array. Preferably, the coated microneedle array has come to a constant weight, indicating that the volatilizable carrier has been removed, before taking the weight after the coating is disposed. Alternatively, the total amount of a solid component (such as the local anesthetic) in the coating on all the microneedles of the entire array can be determined analytically and then the total weight of solids calculated based upon the know weight of all solid components used in the coating formulation.

Volatilizing the carrier can be performed using various means including for example, drying at ambient conditions; drying at conditions other than ambient conditions (such as temperatures other than room temperature or a humidity other than an average humidity); drying for various times; drying with heat, lyophilization, freeze drying; other similar techniques; or combinations thereof.

**FIG. 6** is an optical micrograph illustrating four microneedles of a microneedle array after contacting the microneedles with the composition (coating formulation) and volatilizing the carrier.

Once at least a portion of the carrier (which may be a portion or all of the solvent) in the coating formulation has evaporated (either after a single contacting step or multiple contacting steps), the coating formulation on the microneedle array can be referred to as the "coating" as described above.

Methods of coating microneedle arrays can be used to form coated microneedle arrays. A coating disposed on the microneedles or the coated microneedle array can include a coating on at least a portion of the plurality of microneedles.

As indicated above, a medical device, comprising an array of dissolvable microneedles, a method of extending a topically delivered local anesthetic dose in mammalian tissue using the array of dissolvable microneedles, and a method of making a local anesthetic-containing dissolvable microneedle device are also provided herein. The dissolvable microneedles may contain the same components in the various amounts described above for the coatings disposed on the microneedles.

**FIG. 4** illustrates, in cross-section, a portion of a microneedle array **300** that includes four microneedles **310** (of which two are referenced in **FIG. 4****)** positioned on a microneedle substrate **320.** Dissolvable microneedle portion **360** includes the local anesthetic and dose-extending component and may optionally further contain any of the excipients as described above. The remaining portion of the dissolvable microneedle and substrate **320** comprise a dissolvable matrix material. In order to avoid wasting the local anesthetic and dose-extending component, these materials are preferably located only in portion **360.** However, the local anesthetic and dose-extending component can be included in the entire volume of the microneedles or throughout the entire microneedle array **300,** including the substrate **320.** Preferably, the dissolvable matrix material is included in portion **360** as well as all other portions of the microneedles.

The wt-% of the local anesthetic and dose-extending component in the dissolvable microneedles is based upon the total weight of solids in all portions of the microneedle array that contain these materials. For example, in **FIG. 4****,** the total weight of solids in portion **360** is the basis for the wt-% values.

The dissolvable matrix material may be any solid which dissolves sufficiently in the tissue underlying the stratum corneum to allow the local anesthetic and dose-extending component to be released into the tissue, preferably within 10 minutes, more preferably within 1 minute. For certain embodiments, including any one of the above embodiments which includes dissolvable microneedles, the dissolvable matrix material is selected from the group consisting of hyaluronic acid, carboxymethylcellulose, hydroxpropylmethylcellulose, methylcellulose, polyvinyl alcohol, polyvinyl pyrrolidone, sucrose, glucose, dextran, trehalose, maltodextrin, and a combination thereof.

Dissolvable microneedle arrays may be fabricated by casting and drying a solution containing volatile carrier and dissolvable matrix material (preferably water soluble) in a mold containing the microstructured cavities. The internal shape of the microstructured cavities corresponds to the external shape of the dissolvable microneedles. The mold can be comprised of materials such as polydimethylsiloxane (PDMS) or other plastics that do not permanently bind to or that have low adhesion to materials used to make the dissolvable microneedles.

The local anesthetic and dose-extending component can be incorporated into dissolvable microneedles by first loading a solution of these components with a volatilizable carrier (preferably also including the dissolvable matrix material) into the mold containing microstructured cavities. After at least partially drying (volatilizing at least a portion of the volatilizable carrier), the mold is filled with a solution of dissolvable matrix material (without the anesthetic and dose-extending component), followed by drying. Alternatively, in a one-step process, the local anesthetic and dose-extending component can be combined with the dissolvable matrix material in a solution with the volatilizable carrier, and the mold filled with this solution, followed by drying. The same volatilizable carriers described above in the coating formulations may be used here.

Drying can be carried out using methods such as lyophilization, centrifugation, vacuum, and/or heating. After drying, the solid dissolvable microneedle array is removed from the mold and is ready for use. These solutions may be made using water and/or organic solvents, such as ethanol, as described above to assure solubilization of all materials used in the microneedle array.

Microneedle devices provided herein may be used for immediate delivery, for example, application and immediate removal of the device from the application site. Immediate removal may be within 10 minutes or less, preferably within 1 minute or less.

FIG. 7 is an optical micrograph illustrating coated microneedles provided herein after 1 minute in tissue. It can be clearly seen that most, if not all, of the coating was removed and remained in the tissue.

Application of the microneedle device may be carried out by contacting the tissue of a subject with the microneedles and applying hand pressure to force the microneedles into the tissue. Alternatively, an application device may be used which applies the pressure, forcing the microneedles into the tissue. This can provide a more even distribution of pressure and force the microneedles into the tissue at an optimum velocity so that essentially all of the microneedles can release the local anesthetic and dose-extending component into the tissue. For certain embodiments, including any one of the above embodiments of the method of extending a topically delivered local anesthetic dose in mammalian tissue, contacting the tissue with a microneedle device is carried out at a microneedle velocity of 5 to 10 meters/second.

The following examples are provided to more particularly illustrate various embodiments of the present invention, but the particular materials and amounts thereof recited in these examples, as well as other conditions and details are in no way intended to limit this invention.

### EXAMPLES

All formulations used to coat the microneedle arrays in the following examples were prepared on a weight percent basis (w/w %) and were prepared in water. For example, a formulation comprised of 30% dextran, 20% lidocaine hydrochloride, and 10% tetracaine hydrochloride included 40% water.

### Example 1

### Formulation Containing Lidocaine with Tetracaine

The microneedle arrays were injection molded (3M, St. Paul, MN) from Class VI, medical grade liquid crystalline polymer (LCP) (Vectra ® MT1300, Ticona Plastics, Auburn Hills, Michigan) with a surface area of approximately 1.27cm². Each microneedle array featured 316 four-sided pyramidal-shaped microneedles arranged in an octagonal pattern, with microneedle heights of nominally 500 microns, an aspect ratio of approximately 3:1, and a tip-to-tip distance between neighboring microneedles of nominally 550 microns.

Lidocaine was coated onto the microneedle arrays using a dip-coating process with a formulation comprised of 30% dextran (from Pharmacosmos, Holbaek, Denmark), 20% lidocaine hydrochloride (Sigma, St. Louis, MO) and 10% tetracaine hydrochloride (Spectrum Chemical & Laboratory Products, New Brunswick, NJ). Prior to coating, the microneedle arrays were cleaned with 70% isopropyl alcohol (BDH, West Chester, PA) and dried in a 35°C oven for 1 hr. Microneedle arrays were then dipped into the coating solution once. The coated microneedles were allowed to dry for 1 hr at 35°C. For in vivo application, each array was attached to a 5 cm² adhesive patch with 1513 double-sided medical adhesive (3M Company, St. Paul, MN). The arrays were stored in a light and moisture proof foil pouch (Oliver-Tolas Healthcare Packaging, Feasterville, PA) at room temperature prior to in vivo application.

The determination of lidocaine content in the formulation coated on the microneedles of an array was conducted using an Agilent 1100 HPLC (Agilent Technologies, Wilmington, DE) equipped with a quaternary pump, well-plated thermostatted autosampler, thermostatted column compartment, and diode array UV detector. The formulation coated on the microneedles of an array was desorbed into an appropriate volume of diluent, (0.1% trifluoroacetic acid (TFA, J T. Baker, Phillpsburg, NJ) in water), and injected into the HPLC system. The results were quantified against an external standard of lidocaine (free base) at a similar concentration to the coating amount. A Zorbax SB-C18 column, 3.µm particle size, 150 x 3.0mm I.D. (Agilent Technologies, Wilmington, DE) was used for the separation. The mobile phase consisted of two eluents: eluent A was 100% water with 0.1% TFA and eluent B was 100% acetonitrile (Spectrum Chemical & Laboratory Products, New Brunswick, NJ) with 0.1% TFA. A linear gradient from 80/20 to 0/100 (A/B) was applied over 5 min. The flow rate was 0.5 mL/min and the UV detection wavelength was 230 nm. The total run time was 8 minutes. A total of 5 replicates were conducted. The results from the individual replicates were averaged to provide a measured lidocaine loading amount of 54.4±2.1 mcg/array.

The in vivo delivery of lidocaine to tissue using the coated microneedle array described above was determined using naïve young adult female mixed breed agricultural swine (Yorkshire X from Midwest Research Swine, Gibbon, MN). Swine with minimal skin pigmentation and weighing 10-40 kg were selected for the study. The animals were initially sedated with ketamine (10 mg/kg) and glycopyrrolate (0.011 mg/kg) was intramuscularly administered to reduce salivary, tracheobronchial, and pharyngeal secretions. Hair and dirt on pig skin at the intended application sites were removed prior to application of the microneedle array to minimize complications. Skin test sites were selected based on lack of skin pigmentation and skin damage. The hair was first clipped using an electric shaver followed by shaving with a wet multi-blade disposable razor (Schick Xtreme3) and shaving cream (Gillette Foamy Regular) while the animal was under anesthesia.

A light surgical plane of anesthesia was achieved by administering 1.5-5% isoflurane in 1.5-4 L of oxygen by mask. Anesthetized animals were placed in lateral recumbency on insulated table pads. During the experiment, the animals were placed on a heated table to control body temperature at approximately 38°C. Animals were observed continuously until normal recovery was attained. A microneedle array was applied to the swine rib with a spring-loaded applicator that provided an impact velocity of approximately 8 m/s, held in place with the applicator for 5 seconds before removing the applicator, and remained in contact with the skin for 1 minute. The applicator was previously described in International Publication No. WO 2005/123173 A1. The patch was removed and the application site was swabbed with a cotton ball moistened with phosphate buffered saline (PBS) (EMD chemicals Inc., Gibbstown, NJ) to remove any residual lidocaine remaining on the skin surface. Following this swabbing, a dry cotton ball was used to remove any residual PBS. A 4 mm skin biopsy (Disposable Biopsy Punch from Miltex Inc., York, PA) was collected from the microneedle array application site following removal of the array at time points of 0, 5, 15, 30, 60, 90, and 120 minutes. The biopsy punch samples were stored at -20°C until analyzed.

The animal facility used was accredited by the Association for Assessment and Accreditation of Laboratory Animal Care (AAALAC, Frederick, Maryland) and all procedures were in accordance with an approved Institutional Animal Care and Use Committee (IACUC) protocol.

Lidocaine was extracted from each swine skin tissue biopsy punch using enzymatic digestion. The skin tissue was weighed into a glass vial, then tissue digestion buffer containing 0.1 U proteinase K (EMD Chemicals, San Diego, CA) per mg of skin tissue was added to the vial. The tissue was digested at 55°C for 5 hours. The digestion process produced a homogenous sample solution.

Protein precipitation was used to prepare the digested tissue samples for analysis by LC/MS/MS. Protein was removed from the digested tissue samples by adding 2 volumes of methanol, containing mepivacaine as the internal standard, followed by centrifugation at 14,000 RPM for 10 minutes. The resulting sample was quantitatively analyzed using a Sciex API3000 triple quadrupole mass spectrometer (Applied Biosystems, Foster City, CA) running in positive ion mode using Turbo IonSpray interface to monitor the product ions resulting from the m/z transitions: 235 → 86.2 for lidocaine and 247 → 97.5 for mepivacaine. The linear range for lidocaine was 50.0 to 20,000 ng/mL evaluated using 1/x² curve weighting.

A total of 3 replicates were conducted. The results from the individual replicates were averaged and are presented in Table1.

**Table1. Tissue Concentration of Lidocaine**

| | 0 min | 5 min | 15 min | 30 min | 60 min | 90 min | 120 min |
|---|---|---|---|---|---|---|---|
| Lidocaine Tissue Concentration (ng/mg) | 191.3 | 162.0 | 174.3 | 103.7 | 58.8 | 23.1 | 9.2 |
| Standard Deviation (ng/mg) | 77.4 | 33.2 | 28.1 | 17.7 | 13.0 | 12.5 | 4.2 |

### Example 2

### Formulation Containing Prilocaine with Tetracaine

The microneedle arrays were injection molded (3M, St. Paul, MN) from Class VI, medical grade liquid crystalline polymer (LCP) (Vectra ® MT1300, Ticona Plastics, Auburn Hills, Michigan) with a surface area of approximately 1.27cm². Each microneedle array featured 316 four-sided pyramidal-shaped microneedles arranged in an octagonal pattern, with microneedle heights of nominally 500 microns, an aspect ratio of approximately 3:1, and a tip-to-tip distance between neighboring microneedles of nominally 550 microns.

Prilocaine was coated onto the microneedle arrays using a dip-coating process with a formulation comprised of 30% dextran (from Pharmacosmos, Holbaek, Denmark), 12% prilocaine hydrochloride (Spectrum Chemical & Laboratory Products, New Brunswick, NJ) and 8% tetracaine hydrochloride (Spectrum Chemical & Laboratory Products, New Brunswick, NJ). Prior to coating, the microneedle arrays were cleaned with 70% isopropyl alcohol (BDH, West Chester, PA) and dried in a 35°C oven for 1 hr. Microneedle arrays were then dipped into the coating solution once. The coated microneedles were allowed to dry for 1 hr at 35°C. For in vivo application, each array was attached to a 5 cm² adhesive patch with 1513 double-sided medical adhesive (3M Company, St. Paul, MN). The arrays were stored in a light and moisture proof foil pouch (Oliver-Tolas Healthcare Packaging, Feasterville, PA) at room temperature prior to in vivo application.

The determination of prilocaine content in the formulation coated on the microneedles of an array was conducted using an Agilent 1100 HPLC (Agilent Technologies, Wilmington, DE) equipped with a quaternary pump, well-plated thermostatted autosampler, thermostatted column compartment, and diode array UV detector. The formulation coated on the microneedles of an array was desorbed into an appropriate volume of diluent, (0.1% trifluoroacetic acid (TFA, J T. Baker, Phillpsburg, NJ) in water), and injected into the HPLC system. The results were quantified against an external standard of prilocaine (free base) at a similar concentration to the coating amount. A Zorbax SB-C18 column, 3.5µm particle size, 150 x 3.0mm I.D. (Agilent Technologies, Wilmington, DE) was used for the separation. The mobile phase consisted of two eluents: eluent A was 100% water with 0.1% TFA and eluent B was 100% acetonitrile (Spectrum Chemical & Laboratory Products, New Brunswick, NJ) with 0.1% TFA. A linear gradient from 80/20 to 0/100 (A/B) was applied over 5 min. The flow rate was 0.5 mL/min and the UV detection wavelength was 230 nm. The total run time was 8 minutes. A total of 5 replicates were conducted. The results from the individual replicates were averaged to provide a measured prilocaine loading amount of 50.0±2.2 mcg/array.

The in vivo delivery of prilocaine to tissue using the coated microneedle array described above was determined using naïve young adult female mixed breed agricultural swine (Yorkshire X from Midwest Research Swine, Gibbon, MN). Swine with minimal skin pigmentation and weighing 10-40 kg were selected for the study. The animals were initially sedated with ketamine (10 mg/kg) and glycopyrrolate (0.011 mg/kg) was intramuscularly administered to reduce salivary, tracheobronchial, and pharyngeal secretions. Hair and dirt on pig skin at the intended application sites were removed prior to application of the microneedle array to minimize complications. Skin test sites were selected based on lack of skin pigmentation and skin damage. The hair was first clipped using an electric shaver followed by shaving with a wet multi-blade disposable razor (Schick Xtreme3) and shaving cream (Gillette Foamy Regular) while the animal was under anesthesia.

A light surgical plane of anesthesia was achieved by administering 1.5-5% isoflurane in 1.5-4 L of oxygen by mask. Anesthetized animals were placed in lateral recumbency on insulated table pads. During the experiment, the animals were placed on a heated table to control body temperature at approximately 38°C. Animals were observed continuously until normal recovery was attained. A microneedle array was applied to the swine rib with a spring-loaded applicator that provided an impact velocity of approximately 8 m/s, held in place with the applicator for 5 seconds before removing the applicator, and remained in contact with the skin for 1 minute. The applicator was previously described in International Publication No. WO 2005/123173 A1. The patch was removed and the application site was swabbed with a cotton ball moistened with phosphate buffered saline (PBS) (EMD chemicals Inc., Gibbstown, NJ) to remove any residual prilocaine remaining on the skin surface. Following this swabbing, a dry cotton ball was used to remove any residual PBS. A 4 mm skin biopsy (Disposable Biopsy Punch from Miltex Inc., York, PA) was collected from the microneedle array application site following removal of the array at time points of 0, 5, 15, 30, 60, 90, and 120 minutes. The biopsy punch samples were stored at -20°C until analyzed.

The animal facility used was accredited by the Association for Assessment and Accreditation of Laboratory Animal Care (AAALAC, Frederick, Maryland) and all procedures were in accordance with an approved Institutional Animal Care and Use Committee (IACUC) protocol.

Prilocaine was extracted from each swine skin tissue biopsy punch using enzymatic digestion. The skin tissue was weighed into a glass vial, then tissue digestion buffer containing 0.1 U proteinase K (EMD Chemicals, San Diego, CA) per mg of skin tissue was added to the vial. The tissue was digested at 55°C for 5 hours. The digestion process produced a homogenous sample solution.

Protein precipitation was used to prepare the digested tissue samples for analysis by LC/MS/MS. Protein was removed from the digested tissue samples by adding 2 volumes of methanol, containing mepivacaine as the internal standard, followed by centrifugation at 14,000 RPM for 10 minutes. The resulting sample was quantitatively analyzed using a Sciex API3000 triple quadrupole mass spectrometer (Applied Biosystems, Foster City, CA) running in positive ion mode using Turbo IonSpray interface to monitor the product ions resulting from the m/z transitions: 221.1 → 86.1 for prilocaine and 247 → 97.5 for mepivacaine. The linear range for prilocaine was 50.0 to 20,000 ng/mL evaluated using 1/x² curve weighting.

A total of 3 replicates were conducted. The results from the individual replicates were averaged and are presented in Table 2.

**Table 2. Tissue Concentration of Prilocaine**

| | 0 min | 5 min | 15 min | 30 min | 60 min | 90 min | 120 min |
|---|---|---|---|---|---|---|---|
| Prilocaine Tissue Concentration (ng/mg) | 87.8 | 75.0 | 48.7 | 33.7 | 10.8 | 6.0 | 3.5 |
| Standard Deviation (ng/mg) | 1.5 | 13.4 | 25.0 | 6.6 | 2.5 | 1.4 | 1.7 |

### Example 3

### Formulation Containing Lidocaine with Bupivacaine

The microneedle arrays were injection molded (3M, St. Paul, MN) from Class VI, medical grade liquid crystalline polymer (LCP) (Vectra ® MT1300, Ticona Plastics, Auburn Hills, Michigan) with a surface area of approximately 1.27cm². Each microneedle array featured 316 four-sided pyramidal-shaped microneedles arranged in an octagonal pattern, with microneedle heights of nominally 500 microns, an aspect ratio of approximately 3:1, and a tip-to-tip distance between neighboring microneedles of nominally 550 microns.

Lidocaine was coated onto the microneedle arrays using a dip-coating process with a formulation comprised of 30% dextran (from Pharmacosmos, Holbaek, Denmark), 30% lidocaine hydrochloride (Sigma, St. Louis, MO) and 1.7% bupivacaine hydrochloride (MP Biomedicals LLC, Solon, OH). Prior to coating, the microneedle arrays were cleaned with 70% isopropyl alcohol (BDH, West Chester, PA) and dried in a 35°C oven for 1 hr. Microneedle arrays were then dipped into the coating solution once. The coated microneedles were allowed to dry for 1 hr at 35°C. For in vivo application, each array was attached to a 5 cm² adhesive patch with 1513 double-sided medical adhesive (3M Company, St. Paul, MN). The arrays were stored in a light and moisture proof foil pouch (Oliver-Tolas Healthcare Packaging, Feasterville, PA) at room temperature prior to in vivo application.

The determination of lidocaine content in the formulation coated on the microneedles of an array was conducted using an Agilent 1100 HPLC (Agilent Technologies, Wilmington, DE) equipped with a quaternary pump, well-plated thermostatted autosampler, thermostatted column compartment, and diode array UV detector. The formulation coated on the microneedles of an array was desorbed into an appropriate volume of diluent, (0.1% trifluoroacetic acid (TFA, J T. Baker, Phillpsburg, NJ) in water), and injected into the HPLC system. The results were quantified against an external standard of lidocaine (free base) at a similar concentration to the coating amount. A Zorbax SB-C18 column, 3.5µm particle size, 150 x 3.0mm I.D. (Agilent Technologies, Wilmington, DE) was used for the separation. The mobile phase consisted of two eluents: eluent A was 100% water with 0.1% TFA and eluent B was 100% acetonitrile (Spectrum Chemical & Laboratory Products, New Brunswick, NJ) with 0.1% TFA. A linear gradient from 80/20 to 0/100 (A/B) was applied over 5 min. The flow rate was 0.5 mL/min and the UV detection wavelength was 230 nm. The total run time was 8 minutes. A total of 5 replicates were conducted. The results from the individual replicates were averaged to provide a measured lidocaine loading amount of 91.4±6.0 mcg/array.

The in vivo delivery of lidocaine to tissue using the coated microneedle array described above was determined using naïve young adult female mixed breed agricultural swine (Yorkshire X from Midwest Research Swine, Gibbon, MN). Swine with minimal skin pigmentation and weighing 10-40 kg were selected for the study. The animals were initially sedated with ketamine (10 mg/kg) and glycopyrrolate (0.011 mg/kg) was intramuscularly administered to reduce salivary, tracheobronchial, and pharyngeal secretions. Hair and dirt on pig skin at the intended application sites were removed prior to application of the microneedle array to minimize complications. Skin test sites were selected based on lack of skin pigmentation and skin damage. The hair was first clipped using an electric shaver followed by shaving with a wet multi-blade disposable razor (Schick Xtreme3) and shaving cream (Gillette Foamy Regular) while the animal was under anesthesia.

A light surgical plane of anesthesia was achieved by administering 1.5-5% isoflurane in 1.5-4 L of oxygen by mask. Anesthetized animals were placed in lateral recumbency on insulated table pads. During the experiment, the animals were placed on a heated table to control body temperature at approximately 38°C. Animals were observed continuously until normal recovery was attained. A microneedle array was applied to the swine rib with a spring-loaded applicator that provided an impact velocity of approximately 8 m/s, held in place with the applicator for 5 seconds before removing the applicator, and remained in contact with the skin for 1 minute. The applicator was previously described in International Publication No. WO 2005/123173 A1. The patch was removed and the application site was swabbed with a cotton ball moistened with phosphate buffered saline (PBS) (EMD chemicals Inc., Gibbstown, NJ) to remove any residual lidocaine remaining on the skin surface. Following this swabbing, a dry cotton ball was used to remove any residual PBS. A 4 mm skin biopsy (Disposable Biopsy Punch from Miltex Inc., York, PA) was collected from the microneedle array application site following removal of the array at time points of 0, 5, 15, 30, 60, 90, and 120 minutes. The biopsy punch samples were stored at -20°C until analyzed.

The animal facility used was accredited by the Association for Assessment and Accreditation of Laboratory Animal Care (AAALAC, Frederick, Maryland) and all procedures were in accordance with an approved Institutional Animal Care and Use Committee (IACUC) protocol.

Lidocaine was extracted from each swine skin tissue biopsy punch using enzymatic digestion. The skin tissue was weighed into a glass vial, then tissue digestion buffer containing 0.1 U proteinase K (EMD Chemicals, San Diego, CA) per mg of skin tissue was added to the vial. The tissue was digested at 55°C for 5 hours. The digestion process produced a homogenous sample solution.

Protein precipitation was used to prepare the digested tissue samples for analysis by LC/MS/MS. Protein was removed from the digested tissue samples by adding 2 volumes of methanol, containing mepivacaine as the internal standard, followed by centrifugation at 14,000 RPM for 10 minutes. The resulting sample was quantitatively analyzed using a Sciex API3000 triple quadrupole mass spectrometer (Applied Biosystems, Foster City, CA) running in positive ion mode using Turbo IonSpray interface to monitor the product ions resulting from the m/z transitions: 235 → 86.2 for lidocaine, and 247 → 97.5 for mepivacaine. The linear range for lidocaine was 50.0 to 20,000 ng/mL evaluated using 1/x² curve weighting.

A total of 3 replicates were conducted. The results from the individual replicates were averaged and are presented in Table 3.

**Table 3. Tissue Concentration of Lidocaine**

| | 0 min | 5 min | 15 min | 30 min | 60 min | 90 min | 120 min |
|---|---|---|---|---|---|---|---|
| Lidocaine Tissue Concentration (ng/mg) | 387.0 | 142.0 | 85.2 | 125.3 | 64.0 | 35.4 | 14.8 |
| Standard Deviation (ng/mg) | 47.6 | 21.2 | 19.3 | 10.3 | 12.9 | 8.2 | 4.5 |

### Example 4

### Formulation Containing Lidocaine with Procaine

The microneedle arrays were injection molded (3M, St. Paul, MN) from Class VI, medical grade liquid crystalline polymer (LCP) (Vectra ® MT1300, Ticona Plastics, Auburn Hills, Michigan) with a surface area of approximately 1.27cm². Each microneedle array featured 316 four-sided pyramidal-shaped microneedles arranged in an octagonal pattern, with microneedle heights of nominally 500 microns, an aspect ratio of approximately 3:1, and a tip-to-tip distance between neighboring microneedles of nominally 550 microns.

Lidocaine was coated onto the microneedle arrays using a dip-coating process with a formulation comprised of 30% dextran (from Pharmacosmos, Holbaek, Denmark), 20% lidocaine hydrochloride (Sigma, St. Louis, MO) and 10% procaine hydrochloride (Alfa Aesar, Wardwill, MA). Prior to coating, the microneedle arrays were cleaned with 70% isopropyl alcohol (BDH, West Chester, PA) and dried in a 35°C oven for 1 hr. Microneedle arrays were then dipped into the coating solution once. The coated microneedles were allowed to dry for 1 hr at 35°C. For in vivo application, each array was attached to a 5 cm² adhesive patch with 1513 double-sided medical adhesive (3M Company, St. Paul, MN). The arrays were stored in a light and moisture proof foil pouch (Oliver-Tolas Healthcare Packaging, Feasterville, PA) at room temperature prior to in vivo application.

The determination of lidocaine content in the formulation coated on the microneedles of an array was conducted using an Agilent 1100 HPLC (Agilent Technologies, Wilmington, DE) equipped with a quaternary pump, well-plated thermostatted autosampler, thermostatted column compartment, and diode array UV detector. The formulation coated on the microneedles of an array was desorbed into an appropriate volume of diluent, (0.1% trifluoroacetic acid (TFA, J T. Baker, Phillpsburg, NJ) in water), and injected into the HPLC system. The results were quantified against an external standard of lidocaine (free base) at a similar concentration to the coating amount. A Zorbax SB-C18 column, 3.5µm particle size, 150 x 3.0mm I.D. (Agilent Technologies, Wilmington, DE) was used for the separation. The mobile phase consisted of two eluents: eluent A was 100% water with 0.1% TFA and eluent B was 100% acetonitrile (Spectrum Chemical & Laboratory Products, New Brunswick, NJ) with 0.1% TFA. A linear gradient from 80/20 to 0/100 (A/B) was applied over 5 min. The flow rate was 0.5 mL/min and the UV detection wavelength was 230 nm. The total run time was 8 minutes. A total of 5 replicates were conducted. The results from the individual replicates were averaged to provide a measured lidocaine loading amount of 62.3±2.0 mcg/array.

The in vivo delivery of lidocaine to tissue using the coated microneedle array described above was determined using naïve young adult female mixed breed agricultural swine (Yorkshire X from Midwest Research Swine, Gibbon, MN). Swine with minimal skin pigmentation and weighing 10-40 kg were selected for the study. The animals were initially sedated with ketamine (10 mg/kg) and glycopyrrolate (0.011 mg/kg) was intramuscularly administered to reduce salivary, tracheobronchial, and pharyngeal secretions. Hair and dirt on pig skin at the intended application sites were removed prior to application of the microneedle array to minimize complications. Skin test sites were selected based on lack of skin pigmentation and skin damage. The hair was first clipped using an electric shaver followed by shaving with a wet multi-blade disposable razor (Schick Xtreme3) and shaving cream (Gillette Foamy Regular) while the animal was under anesthesia.

A light surgical plane of anesthesia was achieved by administering 1.5-5% isoflurane in 1.5-4 L of oxygen by mask. Anesthetized animals were placed in lateral recumbency on insulated table pads. During the experiment, the animals were placed on a heated table to control body temperature at approximately 38°C. Animals were observed continuously until normal recovery was attained. A microneedle array was applied to the swine rib with a spring-loaded applicator that provided an impact velocity of approximately 8 m/s, held in place with the applicator for 5 seconds before removing the applicator, and remained in contact with the skin for 1 minute. The applicator was previously described in International Publication No. WO 2005/123173 A1. The patch was removed and the application site was swabbed with a cotton ball moistened with phosphate buffered saline (PBS) (EMD chemicals Inc., Gibbstown, NJ) to remove any residual lidocaine remaining on the skin surface. Following this swabbing, a dry cotton ball was used to remove any residual PBS. A 4 mm skin biopsy (Disposable Biopsy Punch from Miltex Inc., York, PA) was collected from the microneedle array application site following removal of the array at time points of 0, 5, 15, 30, 60, 90, and 120 minutes. The biopsy punch samples were stored at -20°C until analyzed.

The animal facility used was accredited by the Association for Assessment and Accreditation of Laboratory Animal Care (AAALAC, Frederick, Maryland) and all procedures were in accordance with an approved Institutional Animal Care and Use Committee (IACUC) protocol.

Lidocaine was extracted from each swine skin tissue biopsy punch using enzymatic digestion. The skin tissue was weighed into a glass vial, then tissue digestion buffer containing 0.1 U proteinase K (EMD Chemicals, San Diego, CA) per mg of skin tissue was added to the vial. The tissue was digested at 55°C for 5 hours. The digestion process produced a homogenous sample solution.

Protein precipitation was used to prepare the digested tissue samples for analysis by LC/MS/MS. Protein was removed from the digested tissue samples by adding 2 volumes of methanol, containing mepivacaine as the internal standard, followed by centrifugation at 14,000 RPM for 10 minutes. The resulting sample was quantitatively analyzed using a Sciex API3000 triple quadrupole mass spectrometer (Applied Biosystems, Foster City, CA) running in positive ion mode using Turbo IonSpray interface to monitor the product ions resulting from the m/z transitions: 235 → 86.2 for lidocaine, and 247 → 97.5 for mepivacaine. The linear range for lidocaine was 50.0 to 20,000 ng/mL evaluated using 1/x² curve weighting.

A total of 3 replicates were conducted. The results from the individual replicates were averaged and are presented in Table 4.

**Table 4 Tissue Concentration of Lidocaine**

| | 0 min | 5 min | 15 min | 30 min | 60 min | 90 min | 120 min |
|---|---|---|---|---|---|---|---|
| Lidocaine Tissue Concentration (ng/mg) | 271.0 | 207.7 | 119.8 | 122.7 | 53.8 | 42.1 | 29.5 |
| Standard Deviation (ng/mg) | 57.3 | 61.6 | 47.8 | 7.4 | 22.2 | 7.1 | 8.9 |

### Comparative Example 1

### Formulation Containing Lidocaine without Dose-Extending Component

The microneedle arrays were injection molded (3M, St. Paul, MN) from Class VI, medical grade liquid crystalline polymer (LCP) (Vectra ® MT1300, Ticona Plastics, Auburn Hills, Michigan) with a surface area of approximately 1.27cm². Each microneedle array featured 316 four-sided pyramidal-shaped microneedles arranged in an octagonal pattern, with microneedle heights of nominally 500 microns, an aspect ratio of approximately 3:1, and a tip-to-tip distance between neighboring microneedles of nominally 550 microns.

Lidocaine was coated onto the microneedle arrays using a dip-coating process with a formulation comprised of 30% dextran (from Pharmacosmos, Holbaek, Denmark), 30% lidocaine hydrochloride (Sigma, St. Louis, MO). Prior to coating, the microneedle arrays were cleaned with 70% isopropyl alcohol (BDH, West Chester, PA) and dried in a 35°C oven for 1 hr. Microneedle arrays were then dipped into the coating solution once. The coated microneedles were allowed to dry for 1 hr at 35°C. For in vivo application, each array was attached to a 5 cm² adhesive patch with 1513 double-sided medical adhesive (3M Company, St. Paul, MN). The arrays were stored in a light and moisture proof foil pouch (Oliver-Tolas Healthcare Packaging, Feasterville, PA) at room temperature prior to in vivo application.

The determination of lidocaine content in the formulation coated on the microneedles of an array was conducted using an Agilent 1100 HPLC (Agilent Technologies, Wilmington, DE) equipped with a quaternary pump, well-plated thermostatted autosampler, thermostatted column compartment, and diode array UV detector. The formulation coated on the microneedles of an array was desorbed into an appropriate volume of diluent, (0.1% trifluoroacetic acid (TFA, J T. Baker, Phillpsburg, NJ) in water), and injected into the HPLC system. The results were quantified against an external standard of lidocaine (free base) at a similar concentration to the coating amount. A Zorbax SB-C18 column, 3.5µm particle size, 150 x 3.0mm I.D. (Agilent Technologies, Wilmington, DE) was used for the separation. The mobile phase consisted of two eluents: eluent A was 100% water with 0.1% TFA and eluent B was 100% acetonitrile (Spectrum Chemical & Laboratory Products, New Brunswick, NJ) with 0.1% TFA. A linear gradient from 80/20 to 0/100 (A/B) was applied over 5 min. The flow rate was 0.5 mL/min and the UV detection wavelength was 230 nm. The total run time was 8 minutes. A total of 5 replicates were conducted. The results from the individual replicates were averaged to provide a measured lidocaine loading amount of 94.0±9.0 mcg/array.

The in vivo delivery of lidocaine to tissue using the coated microneedle array described above was determined using naïve young adult female mixed breed agricultural swine (Yorkshire X from Midwest Research Swine, Gibbon, MN). Swine with minimal skin pigmentation and weighing 10-40 kg were selected for the study. The animals were initially sedated with ketamine (10 mg/kg) and glycopyrrolate (0.011 mg/kg) was intramuscularly administered to reduce salivary, tracheobronchial, and pharyngeal secretions. Hair and dirt on pig skin at the intended application sites were removed prior to application of the microneedle array to minimize complications. Skin test sites were selected based on lack of skin pigmentation and skin damage. The hair was first clipped using an electric shaver followed by shaving with a wet multi-blade disposable razor (Schick Xtreme3) and shaving cream (Gillette Foamy Regular) while the animal was under anesthesia.

A light surgical plane of anesthesia was achieved by administering 1.5-5% isoflurane in 1.5-4 L of oxygen by mask. Anesthetized animals were placed in lateral recumbency on insulated table pads. During the experiment, the animals were placed on a heated table to control body temperature at approximately 38°C. Animals were observed continuously until normal recovery was attained. A microneedle array was applied to the swine rib with a spring-loaded applicator that provided an impact velocity of approximately 8 m/s, held in place with the applicator for 5 seconds before removing the applicator, and remained in contact with the skin for 1 minute. The applicator was previously described in International Publication No. WO 2005/123173 A1. The patch was removed and the application site was swabbed with a cotton ball moistened with phosphate buffered saline (PBS) (EMD chemicals Inc., Gibbstown, NJ) to remove any residual lidocaine remaining on the skin surface. Following this swabbing, a dry cotton ball was used to remove any residual PBS. A 4 mm skin biopsy (Disposable Biopsy Punch from Miltex Inc., York, PA) was collected from the microneedle array application site following removal of the array at time points of 0, 5, 15, 30, 60, 90, and 120 minutes. The biopsy punch samples were stored at -20°C until analyzed.

The animal facility used was accredited by the Association for Assessment and Accreditation of Laboratory Animal Care (AAALAC, Frederick, Maryland) and all procedures were in accordance with an approved Institutional Animal Care and Use Committee (IACUC) protocol.

Lidocaine was extracted from each swine skin tissue biopsy punch using enzymatic digestion. The skin tissue was weighed into a glass vial, then tissue digestion buffer containing 0.1 U proteinase K (EMD Chemicals, San Diego, CA) per mg of skin tissue was added to the vial. The tissue was digested at 55°C for 5 hours. The digestion process produced a homogenous sample solution.

Protein precipitation was used to prepare the digested tissue samples for analysis by LC/MS/MS. Protein was removed from the digested tissue samples by adding 2 volumes of methanol, containing mepivacaine as the internal standard, followed by centrifugation at 14,000 RPM for 10 minutes. The resulting sample was quantitatively analyzed using a Sciex API3000 triple quadrupole mass spectrometer (Applied Biosystems, Foster City, CA) running in positive ion mode using Turbo IonSpray interface to monitor the product ions resulting from the m/z transitions: 235 → 86.2 for lidocaine and 247 → 97.5 for mepivacaine. The linear range for lidocaine was 50.0 to 20,000 ng/mL evaluated using 1/x² curve weighting.

A total of 3 replicates were conducted. The results from the individual replicates were averaged and are presented in Table 5.

**Table 5. Tissue Concentration of Lidocaine**

| | 0 min | 5 min | 15 min | 30 min | 60 min | 90 min | 120 min |
|---|---|---|---|---|---|---|---|
| Lidocaine Tissue Concentration (ng/mg) | 129.7 | 59.5 | 45.6 | 21.5 | 15.7 | 12.6 | 5.0 |
| Standard Deviation (ng/mg) | 24.7 | 19.2 | 8.8 | 4.9 | 7.6 | 2.9 | 2.0 |

### Comparative Example 2

### Formulation Containing Prilocaine without Dose-Extending Component

The microneedle arrays were injection molded (3M, St. Paul, MN) from Class VI, medical grade liquid crystalline polymer (LCP) (Vectra ® MT1300, Ticona Plastics, Auburn Hills, Michigan) with a surface area of approximately 1.27cm². Each microneedle array featured 316 four-sided pyramidal-shaped microneedles arranged in an octagonal pattern, with microneedle heights of nominally 500 microns, an aspect ratio of approximately 3:1, and a tip-to-tip distance between neighboring microneedles of nominally 550 microns.

Prilocaine was coated onto the microneedle arrays using a dip-coating process with a formulation comprised of 30% dextran (from Pharmacosmos, Holbaek, Denmark), and 15% prilocaine hydrochloride (Spectrum Chemical & Laboratory Products, New Brunswick, NJ). Prior to coating, the microneedle arrays were cleaned with 70% isopropyl alcohol (BDH, West Chester, PA) and dried in a 35°C oven for 1 hr. Microneedle arrays were then dipped into the coating solution once. The coated microneedles were allowed to dry for 1 hr at 35°C. For in vivo application, each array was attached to a 5 cm² adhesive patch with 1513 double-sided medical adhesive (3M Company, St. Paul, MN). The arrays were stored in a light and moisture proof foil pouch (Oliver-Tolas Healthcare Packaging, Feasterville, PA) at room temperature prior to in vivo application.

The determination of prilocaine content in the formulation coated on the microneedles of an array was conducted using an Agilent 1100 HPLC (Agilent Technologies, Wilmington, DE) equipped with a quaternary pump, well-plated thermostatted autosampler, thermostatted column compartment, and diode array UV detector. The formulation coated on the microneedles of an array was desorbed into an appropriate volume of diluent, (0.1% trifluoroacetic acid (TFA, J T. Baker, Phillpsburg, NJ) in water), and injected into the HPLC system. The results were quantified against an external standard of prilocaine (free base) at a similar concentration to the coating amount. A Zorbax SB-C18 column, 3.µm particle size, 150 x 3.0mm I.D. (Agilent Technologies, Wilmington, DE) was used for the separation. The mobile phase consisted of two eluents: eluent A was 100% water with 0.1% TFA and eluent B was 100% acetonitrile (Spectrum Chemical & Laboratory Products, New Brunswick, NJ) with 0.1% TFA. A linear gradient from 80/20 to 0/100 (A/B) was applied over 5 min. The flow rate was 0.5 mL/min and the UV detection wavelength was 230 nm. The total run time was 8 minutes. A total of 5 replicates were conducted. The results from the individual replicates were averaged to provide a measured prilocaine loading amount of 51.3±1.6 mcg/array.

The in vivo delivery of prilocaine to tissue using the coated microneedle array described above was determined using naïve young adult female mixed breed agricultural swine (Yorkshire X from Midwest Research Swine, Gibbon, MN). Swine with minimal skin pigmentation and weighing 10-40 kg were selected for the study. The animals were initially sedated with ketamine (10 mg/kg) and glycopyrrolate (0.011 mg/kg) was intramuscularly administered to reduce salivary, tracheobronchial, and pharyngeal secretions. Hair and dirt on pig skin at the intended application sites were removed prior to application of the microneedle array to minimize complications. Skin test sites were selected based on lack of skin pigmentation and skin damage. The hair was first clipped using an electric shaver followed by shaving with a wet multi-blade disposable razor (Schick Xtreme3) and shaving cream (Gillette Foamy Regular) while the animal was under anesthesia.

A light surgical plane of anesthesia was achieved by administering 1.5-5% isoflurane in 1.5-4 L of oxygen by mask. Anesthetized animals were placed in lateral recumbency on insulated table pads. During the experiment, the animals were placed on a heated table to control body temperature at approximately 38°C. Animals were observed continuously until normal recovery was attained. A microneedle array was applied to the swine rib with a spring-loaded applicator that provided an impact velocity of approximately 8 m/s, held in place with the applicator for 5 seconds before removing the applicator, and remained in contact with the skin for 1 minute. The applicator was previously described in International Publication No. WO 2005/123173 A1. The patch was removed and the application site was swabbed with a cotton ball moistened with phosphate buffered saline (PBS) (EMD chemicals Inc., Gibbstown, NJ) to remove any residual prilocaine remaining on the skin surface. Following this swabbing, a dry cotton ball was used to remove any residual PBS. A 4 mm skin biopsy (Disposable Biopsy Punch from Miltex Inc., York, PA) was collected from the microneedle array application site following removal of the array at time points of 0, 5, 15, 30, 60, 90, and 120 minutes. The biopsy punch samples were stored at -20°C until analyzed.

The animal facility used was accredited by the Association for Assessment and Accreditation of Laboratory Animal Care (AAALAC, Frederick, Maryland) and all procedures were in accordance with an approved Institutional Animal Care and Use Committee (IACUC) protocol.

Prilocaine was extracted from each swine skin tissue biopsy punch using enzymatic digestion. The skin tissue was weighed into a glass vial, then tissue digestion buffer containing 0.1 U proteinase K (EMD Chemicals, San Diego, CA) per mg of skin tissue was added to the vial. The tissue was digested at 55°C for 5 hours. The digestion process produced a homogenous sample solution.

Protein precipitation was used to prepare the digested tissue samples for analysis by LC/MS/MS. Protein was removed from the digested tissue samples by adding 2 volumes of methanol, containing mepivacaine as the internal standard, followed by centrifugation at 14,000 RPM for 10 minutes. The resulting sample was quantitatively analyzed using a Sciex API3000 triple quadrupole mass spectrometer (Applied Biosystems, Foster City, CA) running in positive ion mode using Turbo IonSpray interface to monitor the product ions resulting from the m/z transitions: 221.1 → 86.1 for prilocaine and 247 → 97.5 for mepivacaine. The linear range for prilocaine was 50.0 to 20,000 ng/mL evaluated using 1/x² curve weighting.

A total of 3 replicates were conducted. The results from the individual replicates were averaged and are presented in Table 6.

**Table 6. Tissue Concentration of Prilocaine**

| | 0 min | 5 min | 15 min | 30 min | 60 min | 90 min | 120 min |
|---|---|---|---|---|---|---|---|
| Prilocaine Tissue Concentration (ng/mg) | 79.0 | 48.9 | 41.1 | 16.3 | 7.2 | 6.3 | 2.8 |
| Standard Deviation (ng/mg) | 17.5 | 9.0 | 29.5 | 5.6 | 2.5 | 0.8 | 1.0 |

## Claims

1. A medical device, comprising:
an array of microneedles, and
a coating disposed on the microneedles,
wherein the coating comprises:
a local anesthetic selected from the group consisting of lidocaine, prilocaine, and a combination thereof; and
a local anesthetic dose-extending component selected from the group consisting of tetracaine, ropivacaine, bupivacaine, procaine and a combination thereof;
wherein the local anesthetic is present in an amount of at least 1 wt-% based upon total weight of solids in the coating, and
wherein the local anesthetic and dose-extending component are in a non-eutectic weight ratio.

2. A method of extending a topically delivered local anesthetic dose in mammalian tissue, the method comprising:
contacting the tissue with a local anesthetic-coated microneedle device,
wherein the device comprises:
an array of microneedles, and
a coating disposed on the microneedles,
wherein the coating comprises:
a local anesthetic selected from the group consisting of lidocaine, prilocaine, and a combination thereof; and
a local anesthetic dose-extending component selected from the group consisting of tetracaine, ropivacaine, bupivacaine, procaine and a combination thereof;
wherein the local anesthetic is present in an amount of at least 1 wt-% based upon total weight of solids in the coating, and
wherein the local anesthetic and dose-extending component are in a non-eutectic weight ratio.

3. A method of making a local anesthetic-coated microneedle device comprising:
providing an array of microneedles,
providing a composition comprising:
a local anesthetic selected from the group consisting of lidocaine, prilocaine, and a combination thereof;
a local anesthetic dose-extending component selected from the group consisting of tetracaine, ropivacaine, bupivacaine, procaine and a combination thereof; and
a volatilizable carrier;
contacting the microneedles with the composition, and
volatilizing at least a portion of the carrier to provide a coating disposed on the microneedles;
wherein the coating comprises the local anesthetic in an amount of at least 1 wt-% based upon total weight of solids in the coating, wherein the local anesthetic and dose-extending component are in a non-eutectic weight ratio; and
wherein the device comprises the array of microneedles with the coating disposed on the microneedles.

4. The method of claim 3, wherein the microneedles each have a tip and a base, the tip extending a distance from the base, and contacting is carried out by contacting the tips of the microneedles and a portion of the microneedles extending not more than 90 percent of the distance from the tips to the bases with the composition.

5. The device of claim 1 or the method of any one of claims 2, 3, and 4, wherein the coating further comprises at least one excipient.

6. The device of claim 5 or the method of claim 5, wherein the coating comprises 10 to 75 wt-% of the at least one excipient, based upon total weight of solids in the coating.

7. The device of claim 5 or claim 6 or the method of claim 5 or claim 6, wherein the excipient is selected from the group consisting of sucrose, dextrins, dextrans, hyroxyethyl cellulose (HEC), polyvinyl pyrrolidone (PVP), polyethylene glycols, amino acids, peptides, polysorbate, human serum albumin, saccharin sodium dehydrate, and a combination thereof.

8. The device of any one of claims 5, 6, and 7 or the method of any one of claims 5, 6, and 7, wherein the at least one excipient is a saccharide.

9. The device of claim 8 or the method of claim 8, wherein the saccharide is selected from the group consisting of dextran, sucrose, trehalose, and a combination thereof.

10. The device of any one of claims 1 and 5 through 9 or the method of any one of claims 2 through 9, wherein the local anesthetic is present in an amount of 20 to 90 wt-%, based upon total weight of solids in the coating.

11. The device of any one of claims 1 and 5 through 10 or the method of any one of claims 2 through 10, wherein the dose-extending component is present in an amount of 2 to 48 wt-%, based upon total weight of solids in the coating.

12. The device of any one of claims 1 and 5 through 11 or the method of any one of claims 2 through 11, wherein the coating is present on the microneedles in an average amount of 0.01 to 2 micrograms per microneedle.

13. The device of any one of claims 1 and 5 through 12 or the method of any one of claims 2 through 12, wherein the microneedles have a height of 200 to 1000 micrometers.

14. The device of claim 13 or the method of claim 13, wherein at least 50 % of the microneedles have the coating present on the microneedles near the tip and extending not more than 50 percent of the distance toward the base.

15. A medical device, comprising an array of dissolvable microneedles, the microneedles comprising:
a dissolvable matrix material;
at least 1 wt-% of a local anesthetic selected from the group consisting of lidocaine, prilocaine, and a combination thereof; and
a local anesthetic dose-extending component selected from the group consisting of tetracaine, ropivacaine, bupivacaine, procaine and a combination thereof;
wherein the local anesthetic and dose-extending component are in a non-eutectic weight ratio, and
wherein wt-% is based upon total weight of solids in all portions of the dissolvable microneedles which contain the local anesthetic.

## Patentansprüche

1. Medizinische Vorrichtung, umfassend:
eine Anordnung von Mikronadeln und
eine auf den Mikronadeln aufgebrachte Beschichtung,
wobei die Beschichtung Folgendes umfasst:
ein Lokalanästhetikum, ausgewählt aus der Gruppe bestehend aus Lidocain, Prilocain und einer Kombination davon, und
einen die Dosis des Lokalanästhetikums ausdehnenden Bestandteil, ausgewählt aus der Gruppe bestehend aus Tetracain, Ropivacain, Bupivacain, Procain und einer Kombination davon,
wobei das Lokalanästhetikum in einer Menge von mindestens 1 Gew.-% basierend auf dem Gesamtgewicht der Feststoffe in der Beschichtung vorliegt und
wobei das Lokalanästhetikum und der die Dosis ausdehnende Bestandteil in einem nicht-eutektischen Gewichtsverhältnis vorliegen.

2. Verfahren zum Ausdehnen einer Dosis eines in Säugetiergewebe topisch verabreichten Lokalanästhetikums, wobei das Verfahren Folgendes umfasst:
Inkontaktbringen des Gewebes mit einer mit einem Lokalanästhetikum beschichteten Mikronadelvorrichtung,
wobei die Vorrichtung Folgendes umfasst:
eine Anordnung von Mikronadeln und
eine auf den Mikronadeln aufgebrachte Beschichtung,
wobei die Beschichtung Folgendes umfasst:
ein Lokalanästhetikum, ausgewählt aus der Gruppe bestehend aus Lidocain, Prilocain und einer Kombination davon, und
einen die Dosis des Lokalanästhetikums ausdehnenden Bestandteil, ausgewählt aus der Gruppe bestehend aus Tetracain, Ropivacain, Bupivacain, Procain und einer Kombination davon,
wobei das Lokalanästhetikum in einer Menge von mindestens 1 Gew.-% basierend auf dem Gesamtgewicht der Feststoffe in der Beschichtung vorliegt, und
wobei das Lokalanästhetikum und der die Dosis ausdehnende Bestandteil in einem nicht-eutektischen Gewichtsverhältnis vorliegen.

3. Verfahren zur Herstellung einer mit Lokalanästhetikum beschichteten Mikronadelvorrichtung, umfassend:
Bereitstellen einer Anordnung von Mikronadeln,
Bereitstellen einer Zusammensetzung, umfassend:
ein Lokalanästhetikum, ausgewählt aus der Gruppe bestehend aus Lidocain, Prilocain und einer Kombination davon,
einen die Dosis des Lokalanästhetikums ausdehnenden Bestandteil, ausgewählt aus der Gruppe bestehend aus Tetracain, Ropivacain, Bupivacain, Procain und einer Kombination davon, und
einen verdampfbaren Träger,
Inkontaktbringen der Mikronadeln mit der Zusammensetzung und
Verdampfen mindestens eines Teils des Trägers, um eine auf den Mikronadeln aufgebrachte Beschichtung bereitzustellen,
wobei die Beschichtung das Lokalanästhetikum in einer Menge von mindestens 1 Gew.-% basierend auf dem Gesamtgewicht der Feststoffe in der Beschichtung umfasst, wobei das Lokalanästhetikum und der die Dosis ausdehnende Bestandteil in einem nicht-eutektischen Gewichtsverhältnis vorliegen, und
wobei die Vorrichtung die Anordnung von Mikronadeln mit der auf den Mikronadeln aufgebrachten Beschichtung umfasst.

4. Verfahren nach Anspruch 3, wobei die Mikronadeln jeweils eine Spitze und eine Basis aufweisen, wobei sich die Spitze in einem Abstand von der Basis erstreckt und das Inkontaktbringen durch Inkontaktbringen der Spitzen der Mikronadeln und eines Abschnitts der Mikronadeln, der sich nicht weiter als 90 Prozent des Abstands der Spitzen von den Basen erstreckt, mit der Zusammensetzung erfolgt.

5. Vorrichtung nach Anspruch 1 oder Verfahren nach einem der Ansprüche 2, 3 und 4, wobei die Beschichtung ferner mindestens einen Arzneimittelträger umfasst.

6. Vorrichtung nach Anspruch 5 oder Verfahren nach Anspruch 5, wobei die Beschichtung zu 10 bis 75 Gew.-% den mindestens einen Arzneimittelträger basierend auf dem Gesamtgewicht der Feststoffe in der Beschichtung umfasst.

7. Vorrichtung nach Anspruch 5 oder Anspruch 6 oder Verfahren nach Anspruch 5 oder Anspruch 6, wobei der Arzneimittelträger ausgewählt ist aus der Gruppe bestehend aus Saccharose, Dextrinen, Dextranen, Hydroxyethylcellulose (HEC), Polyvinylpyrrolidon (PVP), Polyethylenglykolen, Aminosäuren, Peptiden, Polysorbat, menschlichem Serumalbumin, Saccharinnatriumdihydrat und einer Kombination davon.

8. Vorrichtung nach einem der Ansprüche 5, 6 und 7 oder Verfahren nach einem der Ansprüche 5, 6 und 7, wobei es sich bei dem mindestens einen Arzneimittelträger um ein Saccharid handelt.

9. Vorrichtung nach Anspruch 8 oder Verfahren nach Anspruch 8, wobei das Saccharid ausgewählt ist aus der Gruppe bestehend aus Dextran, Saccharose, Trehalose und einer Kombination davon.

10. Vorrichtung nach einem der Ansprüche 1 und 5 bis 9 oder Verfahren nach einem der Ansprüche 2 bis 9, wobei das Lokalanästhetikum in einer Menge von 20 bis 90 Gew.-% basierend auf dem Gesamtgewicht der Feststoffe in der Beschichtung vorliegt.

11. Vorrichtung nach einem der Ansprüche 1 und 5 bis 10 oder Verfahren nach einem der Ansprüche 2 bis 10, wobei der die Dosis ausdehnende Bestandteil in einer Menge von 2 bis 48 Gew.-% basierend auf dem Gesamtgewicht der Feststoffe in der Beschichtung vorliegt.

12. Vorrichtung nach einem der Ansprüche 1 und 5 bis 11 oder Verfahren nach einem der Ansprüche 2 bis 11, wobei die Beschichtung auf den Mikronadeln in einer durchschnittlichen Menge von 0,01 bis 2 Mikrogramm pro Mikronadel vorliegt.

13. Vorrichtung nach einem der Ansprüche 1 und 5 bis 12 oder Verfahren nach einem der Ansprüche 2 bis 12, wobei die Mikronadeln eine Höhe von 200 bis 1000 Mikrometern aufweisen.

14. Vorrichtung nach Anspruch 13 oder Verfahren nach Anspruch 13, wobei mindestens 50 % der Mikronadeln die Beschichtung in der Nähe der Spitze und sich über nicht mehr als 50 Prozent des Abstands zur Basis erstreckend aufweisen.

15. Medizinische Vorrichtung, umfassend eine Anordnung von auflösbaren Mikronadeln, wobei die Mikronadeln Folgendes umfassen:
ein auflösbares Matrixmaterial,
zu mindestens 1 Gew.-% ein Lokalanästhetikum, ausgewählt aus der Gruppe bestehend aus Lidocain, Prilocain und Kombinationen davon, und
einen die Dosis des Lokalanästhetikums ausdehnenden Bestandteil,
ausgewählt aus der Gruppe bestehend aus Tetracain, Ropivacain, Bupivacain, Procain und einer Kombination davon,
wobei das Lokalanästhetikum und der die Dosis ausdehnende Bestandteil in einem nicht-eutektischen Gewichtsverhältnis vorliegen,
und
wobei Gew.-% auf dem Gesamtgewicht der Feststoffe in allen Abschnitten der auflösbaren Mikronadeln, die das Lokalanästhetikum enthalten, basiert.

## Revendications

1. Dispositif médical, comprenant :
un réseau de micro-aiguilles, et
un revêtement disposé sur les micro-aiguilles,
dans lequel le revêtement comprend :
un anesthésique local choisi parmi le groupe constitué de lidocaïne, de prilocaïne et d'une combinaison de celles-ci ; et
un composant d'extension de dose anesthésique local choisi parmi le groupe constitué de tétracaïne, de ropivacaïne, de bupivacaïne, de procaïne et d'une combinaison de celles-ci ;
dans lequel l'anesthésique local est présent en une quantité d'au moins 1 % en poids sur base du poids total de solides dans le revêtement, et
dans lequel l'anesthésique local et le composant d'extension de dose sont dans un rapport pondéral non eutectique.

2. Procédé d'extension d'une dose d'anesthésique local libérée localement dans un tissu mammalien, le procédé comprenant :
la mise en contact du tissu avec un dispositif à micro-aiguilles revêtues d'anesthésique local,
dans lequel le dispositif comprend :
un réseau de micro-aiguilles, et
un revêtement disposé sur les micro-aiguilles,
dans lequel le revêtement comprend :
un anesthésique local choisi parmi le groupe constitué de lidocaïne, de prilocaïne et d'une combinaison de celles-ci ; et
un composant d'extension de dose d'anesthésique local choisi parmi le groupe constitué de tétracaïne, de ropivacaïne, de bupivacaïne, de procaïne et d'une combinaison de celles-ci ;
dans lequel l'anesthésique local est présent en une quantité d'au moins 1 % en poids sur base du poids total de solides dans le revêtement, et
dans lequel l'anesthésique local et le composant d'extension de dose sont dans un rapport pondéral non eutectique.

3. Procédé de fabrication d'un dispositif à micro-aiguilles revêtues d'anesthésique local comprenant :
la fourniture d'un réseau de micro-aiguilles,
la fourniture d'une composition comprenant :
un anesthésique local choisi parmi le groupe constitué de lidocaïne, de prilocaïne et d'une combinaison de celles-ci ;
un composant d'extension de dose d'anesthésique local choisi parmi le groupe constitué de tétracaïne, de ropivacaïne, de bupivacaïne, de procaïne et d'une combinaison de celles-ci ; et
un support volatilisable ;
la mise en contact des micro-aiguilles avec la composition, et
la volatilisation d'au moins une partie du support pour fournir un revêtement disposé sur les micro-aiguilles ;
dans lequel le revêtement comprend l'anesthésique local en une quantité d'au moins 1 % en poids sur base du poids total de solides dans le revêtement, dans lequel l'anesthésique local et le composant d'extension de dose sont dans un rapport pondéral non eutectique ; et
dans lequel le dispositif comprend le réseau de micro-aiguilles avec le revêtement disposé sur les micro-aiguilles.

4. Procédé selon la revendication 3, dans lequel les micro-aiguilles ont chacune une pointe et une base, la pointe s'étendant à une certaine distance de la base, et la mise en contact est effectuée par la mise en contact des pointes des micro-aiguilles et d'une partie des micro-aiguilles ne s'étendant pas à plus de 90 pour cent de la distance allant des pointes aux bases avec la composition.

5. Dispositif selon la revendication 1 ou procédé selon l'une quelconque des revendications 2, 3 et 4, dans lequel le revêtement comprend en outre au moins un excipient.

6. Dispositif selon la revendication 5 ou procédé selon la revendication 5, dans lequel le revêtement comprend 10 à 75 % en poids de l'au moins un excipient, sur base du poids total de solides dans le revêtement.

7. Dispositif selon la revendication 5 ou la revendication 6 ou procédé selon la revendication 5 ou la revendication 6, dans lequel l'excipient est choisi parmi le groupe constitué de saccharose, de dextrines, de dextranes, d'hydroxyéthylcellulose (HEC), de polyvinylpyrrolidone (PVP), de polyéthylène glycols, d'acides aminés, de peptides, de polysorbate, de sérumalbumine humaine, de saccharine sodique déshydratée, et d'une combinaison de ceux-ci.

8. Dispositif selon l'une quelconque des revendications 5, 6 et 7 ou procédé selon l'une quelconque des revendications 5, 6 et 7, dans lequel l'au moins un excipient est un saccharide.

9. Dispositif selon la revendication 8 ou procédé selon la revendication 8, dans lequel le saccharide est choisi parmi le groupe constitué de dextrane, de saccharose, de tréhalose et d'une combinaison de ceux-ci.

10. Dispositif selon l'une quelconque des revendications 1 et 5 à 9 ou procédé selon l'une quelconque des revendications 2 à 9, dans lequel l'anesthésique local est présent en une quantité de 20 à 90 % en poids, sur base du poids total de solides dans le revêtement.

11. Dispositif selon l'une quelconque des revendications 1 et 5 à 10 ou procédé selon l'une quelconque des revendications 2 à 10, dans lequel le composant d'extension de dose est présent en une quantité de 2 à 48 % en poids, sur base du poids total de solides dans le revêtement.

12. Dispositif selon l'une quelconque des revendications 1 et 5 à 11 ou procédé selon l'une quelconque des revendications 2 à 11, dans lequel le revêtement est présent sur les micro-aiguilles en une quantité moyenne de 0,01 à 2 microgrammes par micro-aiguille.

13. Dispositif selon l'une quelconque des revendications 1 et 5 à 12 ou procédé selon l'une quelconque des revendications 2 à 12, dans lequel les micro-aiguilles ont une hauteur de 200 à 1000 micromètres.

14. Dispositif selon la revendication 13 ou procédé selon la revendication 13, dans lequel au moins 50 % des micro-aiguilles ont le revêtement présent sur les micro-aiguilles près de la pointe et ne s'étendant pas sur plus de 50 pour cent de la distance en direction de la base.

15. Dispositif médical, comprenant un réseau de micro-aiguilles solubles, les micro-aiguilles comprenant :
un matériau de matrice soluble ;
au moins 1 % en poids d'un anesthésique local choisi parmi le groupe constitué de lidocaïne, de prilocaïne et d'une combinaison de celles-ci ; et
un composant d'extension de dose d'anesthésique local choisi parmi le groupe constitué de tétracaïne, de ropivacaïne, de bupivacaïne, de procaïne et d'une combinaison de celles-ci ;
dans lequel l'anesthésique local et le composant d'extension de dose sont dans un rapport pondéral non eutectique, et
dans lequel le % en poids est sur base du poids total de solides dans toutes les parties des micro-aiguilles solubles qui contiennent l'anesthésique local.
